# EUROPÄISCHE PATENTSCHRIFT

(11) **EP 2 288 597 B1**
(45) Veröffentlichungstag und Bekanntmachung des Hinweises auf die Patenterteilung: **29.10.2014**
(21) Anmeldenummer: 09742026.9
(22) Anmeldetag: 04.05.2009
(51) Int. Cl.: C07D 231/14, C07D 409/12, C07C 251/76, C07C 251/84, C07C 251/86

(54) **VERFAHREN ZUR HERSTELLUNG VON 1,3,4-SUBSTITUIERTEN PYRAZOLVERBINDUNGEN**
METHOD FOR PREPARING 1,3,4-SUBSTITUTED PYRAZOL COMPOUNDS
PROCÉDÉ DE PRODUCTION DE COMPOSÉS PYRAZOLIQUES 1,3,4-SUBSTITUÉS

(30) Priorität: 05.05.2008 EP 08155657
(43) Veröffentlichungstag der Anmeldung: 02.03.2011
(73) Patentinhaber: BASF SE, 67056 Ludwigshafen (DE)
(72) Erfinder: WOLF, Bernd, 67136 Fußgönheim (DE); MAYWALD, Volker, 67071 Ludwigshafen (DE); KEIL, Michael, 67251 Freinsheim (DE); KORADIN, Christopher, 67067 Ludwigshafen (DE); RACK, Michael, 69214 Eppelheim (DE); ZIERKE, Thomas, 67459 Böhl-Iggelheim (DE); SUKOPP, Martin, 68165 Mannheim (DE)
(86) Internationale Anmeldenummer: PCT/EP2009/055328
(87) Internationale Veröffentlichungsnummer: WO 2009/135808

(56) Entgegenhaltungen:
- EP-A- 0 581 725
- WO-A-2005/042468
- US-A- 5 498 624
- ETSUJI OKADA ET AL: "Facile synthetic methods for 3- and 5-trifluoromethyl- 4-trifluoroacetyl-pyrazoles and their conversion into pyrazole-4-carboxylic acids" HETEROCYCLES, ELSEVIER SCIENCE PUBLISHERS B.V. AMSTERDAM, NL, Bd. 34, Nr. 4, 1. Januar 1992 (1992-01-01), Seiten 791-798, XP009103121 ISSN: 0385-5414
- VINOGRADOVA N B ET AL: "Synthesis and Mechanism of the Formation of Bis(methylamides) of Pyrazoledicarboxylic Acids" HIMIA GETEROSCIKLICESKIH SOEDINENIJ - CHEMISTRY OF HETEROCYCLIC COMPOUNDS, LATVIJSKIJ INSTITUT ORGANICESKOGO SINTEZA, RIGA, LV, Bd. 4, 1. Januar 1968 (1968-01-01), Seiten 502-507, XP002488451 ISSN: 0132-6244
- ALTENBACH R J ET AL: "Synthesis, Potency, and in vivo Profiles of Quinoline containing Histamine H3 Receptor Inverse Agonists" JOURNAL OF MEDICINAL CHEMISTRY, US AMERICAN CHEMICAL SOCIETY. WASHINGTON, Bd. 50, Nr. 22, 1. November 2007 (2007-11-01), Seiten 5439-5448, XP002488452 ISSN: 0022-2623 [gefunden am 2007-10-06]

## Beschreibung

Die vorliegende Erfindung betrifft ein Verfahren zur Herstellung von 1,3,4-substituierten Pyrazolverbindungen der Formel I, 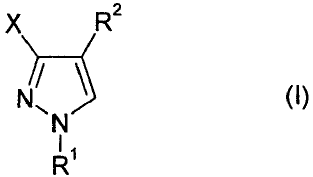 worin
- X: für eine Gruppe CX¹X²X³ steht, worin
- X¹, X² und X³: unabhängig voneinander für Wasserstoff, Fluor oder Chlor stehen, wobei X¹ auch für C₁-C₆-Alkyl oder C₁-C₄-Haloalkyl stehen kann und wobei wenigstens einer der Reste X¹, X² von Wasserstoff verschieden ist,
- R¹: für Methyl steht, und
- R²: für eine Gruppe CO₂R^{2a} steht, worin
- R^{2a}: für C₅-C₆-Cycloalkyl, gegebenenfalls substituiertes Phenyl oder C₁-C₆-Alkyl steht, das gegebenenfalls durch C₁-C₄-Alkoxy, Phenyl oder C₃-C₆-Cycloalkyl substituiert sein kann.

Pyrazole der allgemeinen Formel I sind wichtige Ausgangsprodukte für eine Reihe von pharmazeutischen Wirkstoffen und Pflanzenschutzwirkstoffen, insbesondere von 1,3-substituierten Pyrazol-4-ylcarbonsäureaniliden, wie sie beispielsweise in US 5,498,624, EP 545099 A1, EP 589301 A1, WO 92/12970, WO 03/066610, WO 2006/024389, WO 2007/003603, WO 2007/006806 beschrieben werden.

Die Herstellung 1,3,4-substituierter Pyrazolverbindungen der Formel I erfolgt typischerweise durch Cyclisierung geeigneter 1,3-difunktioneller Verbindungen mit substituierten Hydrazinverbindungen oder durch Umsetzung 1,3-difunktioneller Verbindungen mit Hydrazin, gefolgt von einer Alkylierung zur Einführung des Substituenten am Stickstoff (1-Position). Ein prinzipieller Nachteil bei dieser Vorgehensweise ist die mangelnde Regioselektivität der Cyclisierung 1,3-difunktioneller Verbindungen mit substituierten Hydrazinverbindungen wie auch die mangelnde Regioselektivität der N-Alkylierung von Pyrazolen, so dass in beiden Fällen neben der gewünschten 1,3,4-substituierten Pyrazolverbindung der Formel I (1,3-Isomer) auch das 1,4,5-substituierte Isomer der Formel I' (1,5-Isomer) gebildet wird.

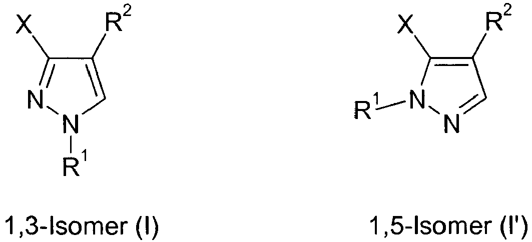

Abgesehen davon, dass die mangelnde Selektivität zu Ausbeuteverlusten führt, lassen sich 1,3-Isomer der Formel I und 1,5-Isomer der Formel I' häufig nur schlecht trennen. Um akzeptable Selektivitäten zu erzielen, müssen die Reaktionen daher bei tiefen Temperaturen durchgeführt werden, wodurch sich der apparative Aufwand beträchtlich erhöht. Zudem ist auch in der Kälte die Regioselektivität nicht vollständig zufriedenstellend.

Die US 5,498,624 u. A. beschreiben ein Verfahren zur Herstellung von (3-Difluormethyl-1-methylpyrazol-4-yl)carbonsäureestern, bei dem man α-Ethoxymethylen-4,4-difluor-3-oxobuttersäureester mit Methylhydrazin zur Pyrazolverbindung cyclisiert. Aus WO 92/12970 ist ein vergleichbares Verfahren bekannt, bei dem 4,4-Difluor-3-oxo-buttersäurester sukzessive mit Triethylorthoformiat und mit Methylhydrazin umsetzt, wobei intermediär Ethoxymethylen-4,4-difluor-3-oxobuttersäureester entsteht. Die Selektivität bezüglich des gewünschten Isomers ist nicht zufriedenstellend.

Die WO 2003/051820 und WO 2005/042468 beschreiben die Cyclisierung von 2-Halogenacyl-3-aminoacrylsäureestern mit Alkylhydrazinen zu 1-Alkyl-3-halogenalkylpyrazol-4-carbonsäureestern. Die Selektivität bezüglich des gewünschten Isomers ist nicht zufriedenstellend.

Die WO 2008/022777 beschreibt ein Verfahren zur Herstellung von 1-substituierten 3-(Dihalogenmethyl)pyrazol-4-carbonsäureestern, bei dem man vinyloge Amidiniumsalze, die durch Umsetzung von α-(Halogenmethyl)-difluormethylaminen mit Acrylaten in Gegenwart einer Lewis-Säure erhältlich sind, mit substituierten Hydrazinen umsetzt. Die Selektivität bezüglich des gewünschten Isomers ist nicht zufriedenstellend.

Der Erfindung liegt daher die Aufgabe zugrunde, ein Verfahren zur Herstellung von 1,3,4-substituierten Pyrazolverbindungen der eingangs zitierten Formel I bereitzustellen, das das gewünschte 1,3-Isomer der Formel I mit hohen Ausbeuten und guter Selektivität liefert.

Es wurde überraschenderweise gefunden, dass man 1,3,4-substituierte Pyrazolverbindungen der eingangs definierten Formel I in einfacher Weise mit hohen Ausbeuten und hoher Regioselektivität bezüglich des gewünschten 1,3-Isomers herstellen kann, wenn man geeignete 1,3-difunktionelle Verbindungen der im folgenden beschriebenen Formel II zunächst mit einem Hydrazon der im folgenden beschriebenen Formel III umsetzt und das dabei gebildete Zwischenprodukt mit einer Säure in Gegenwart von Wasser behandelt.

Dementsprechend betrifft die vorliegende Erfindung ein Verfahren zur Herstellung von 1,3-substituierten Pyrazolverbindungen der eingangs definierten Formel I, welches die folgenden Schritte umfasst:
i) Umsetzung eine Verbindung der Formel II mit einem Hydrazon der Formel III, 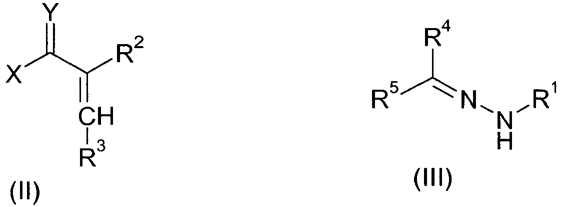 wobei in Formel II die Variablen X und R² die für Formel I angegebenen Bedeutungen aufweisen,
   - Y: für Sauerstoff, eine Gruppe NR^{y1} oder eine Gruppe [NR^{y2}R^{y3}]⁺Z⁻ steht, worin R^{y1}, R^{y2} und R^{y3} unabhängig voneinander für C₁-C₆-Alkyl, C₅-C₆-Cycloalkyl, gegebenenfalls substituiertes Phenyl oder gegebenenfalls substituiertes Phenyl-C₁-C₄-alkyl stehen, oder
   R^{y2} und R^{y3} zusammen mit dem Stickstoffatom, an das diese gebunden sind, für einen N-gebundenen 5- bis 8-gliedrigen, gesättigten, gegebenenfalls substituierten Heterocyclus stehen, der neben dem Stickstoffatom noch 1 oder 2 weitere Heteroatome ausgewählt unter N, O und S als Ringatome aufweisen kann, und Z- für einen Anion steht;
   - R³: für OR^{3a} oder eine Gruppe NR^{3b}R^{3c} steht, worin
   R^{3a}, R^{3b} und R^{3c} unabhängig voneinander für C₁-C₆-Alkyl, C₅-C₆-Cycloalkyl, gegebenenfalls substituiertes Phenyl oder gegebenenfalls substituiertes Phenyl-C₁-C₄-alkyl stehen, stehen, oder
   R^{3b} und R^{3c} zusammen mit dem Stickstoffatom, an das diese gebunden sind, für einen N-gebundenen 5- bis 8-gliedrigen, gesättigten, gegebenenfalls substituierten Heterocyclus stehen, der neben dem Stickstoffatom noch 1 oder 2 weitere Heteroatome ausgewählt unter N, O und S als Ringatome aufweisen kann,
   und wobei in Formel III die Variable R¹ die für Formel I angegebenen Bedeutungen aufweist,
   - R⁴ und R⁵: unabhängig voneinander für Wasserstoff, C₁-C₆-Alkyl, das gegebenenfalls durch C₁-C₄-Alkoxy, Phenyl oder C₃-C₆-Cycloalkyl substituiert sein kann, C₃-C₆-Cycloalkyl oder gegebenenfalls substituiertes Phenyl stehen, wobei wenigstens einer der Reste R⁴ oder R⁵ von Wasserstoff verschieden ist, und wobei
   - R⁴ und R⁵: gemeinsam mit dem Kohlenstoffatom, an das sie gebunden sind, auch für einen 5- bis 10-gliedrigen gesättigten Carbocyclus stehen können, der gegebenenfalls ein- oder mehrfach durch C₁-C₄-Alkylgruppen und/oder gegebenenfalls substituiertes Phenyl substituiert ist und/oder einen oder 2 kondensierte Phenylringe aufweist;
ii) Behandlung des dabei erhaltenen Reaktionsprodukts mit einer Säure in Gegenwart von Wasser.

Das erfindungsgemäße Verfahren ist mit einer Reihe von Vorteilen verbunden. Zum einen liefert es die gewünschten 1,3,4-substituierten Pyrazole mit hoher Ausbeute und hoher Regioselektivität bezüglich des gewünschten 1,3-Isomers der Formel I. Zudem sind zur Erreichung der gewünschten Selektivität keine tiefen Temperaturen erforderlich und Schritt i) sowie Schritt ii) können bei moderaten Temperaturen, z.B. im Bereich von 10 bis 180°C, insbesondere im Bereich von 20 bis 150°C durchgeführt werden. Selbstverständlich kann die Umsetzung in den Schritten i) und ii) auch bei geringeren Temperaturen z.B. bei Temperaturen bis -20°C durchgeführt werden, was zur Erzielung der gewünschten Regioselektivität jedoch nicht erforderlich ist.

In Schritt i) des erfindungsgemäßen Verfahrens wird die Verbindung der im Folgenden gezeigten Formel VI gebildet, die sich üblicherweise isolieren lässt:

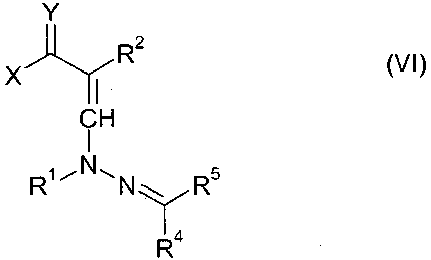

In Formel VI haben X, Y, R¹, R², R⁴ und R⁵ die hier und im Folgenden angegebenen Bedeutungen. Die Verbindungen der Formel VI sind neu, ausgenommen Verbindungen der Formel VI, worin R⁴ und R⁵ jeweils für gegebenenfalls substituiertes Phenyl stehen und Y Sauerstoff bedeutet. Letztere sind aus der EP 581725 bekannt. Die neuen Verbindungen der Formel VI sind ebenfalls Gegenstand der vorliegenden Erfindung.

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen. Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder lod, speziell Fluor, Chlor oder Brom.

### Beispiele anderer Bedeutungen sind:

Der Begriff "C₁-C₆-Alkyl", wie hierin verwendet, bezeichnet eine gesättigte, geradkettige oder verzweigte Kohlenwasserstoffgruppe, umfassend 1 bis 6 Kohlenstoffatome, speziell 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

Der Begriff "C₁-C₆-Alkyl", das gegebenenfalls durch C₁-C₄-Alkoxy, Phenyl oder C₃-C₆-Cycloalkyl substituiert sein kann, steht für unsubstituiertes C₁-C₆-Alkyl, wie zuvor definiert, oder für C₁-C₆-Alkyl, worin eines der Wasserstoffatome durch eine C₁-C₄-Alkoxy-, Phenyl- oder C₃-C₆-Cycloalkylgruppe ersetzt ist.

Der Begriff "C₁-C₄-Haloalkyl", wie hierin verwendet, beschreibt geradkettige oder verzweigte Alkylgruppen mit 1 bis 4 Kohlenstoffatomen, worin die Wasserstoffatome dieser Gruppen teilweise oder vollständig durch Halogenatome, insbesondere durch Fluor und/oder Chlor ersetzt sind, beispielsweise Chlormethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl etc.

Der Begriff "C₁-C₆-Alkoxy", wie hierin verwendet, beschreibt geradkettige oder verzweigte gesättigte Alkylgruppen, umfassend 1 bis 6 Kohlenstoffatome, die über ein Sauerstoffatom gebunden sind. Beispiele umfassen C₁-C₆-Alkoxy wie beispielsweise Methoxy, Ethoxy, OCH₂-C₂H₅, OCH(CH₃)₂, n-Butoxy, OCH(CH₃)-C₂H₅, OCH₂-CH(CH₃)₂, OC(CH₃)₃, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, etc.

Der Begriff "C₁-C₄-Alkoxy-C₁-C₆-alkyl", wie hierin verwendet, beschreibt C₁-C₆-Alkyl, worin eines der Wasserstoffatome durch eine C₁-C₄-Alkoxygruppe ersetzt ist. Beispiele hierfür sind CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethyl-ethoxy)ethyl, 2-(Methoxy)propyl, 2-(Ethoxy)propyl, 2-(n-Propoxy)propyl, 2-(1-Methyl-ethoxy)propyl, 2-(n-Butoxy)propyl, 2-(1-Methylpropoxy)propyl, 2-(2-Methylpropoxy)-propyl, 2-(1,1-Dimethylethoxy)propyl, 3-(Methoxy)propyl, 3-(Ethoxy)propyl, 3-(n-Prop-oxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)-butyl, 2-(n-Propoxy)butyl, 2-(1-Methylethoxy)butyl, 2-(n-Butoxy)butyl, 2-(1-Methylprop-oxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl, 4-(1,1-Dimethyl-ethoxy)butyl, etc.

Der Begriff "C₃-C₆-Cycloalkyl" wie hierin verwendet, beschreibt monocyclische, gesättigte Kohlenwasserstoffradikale, umfassend 3 bis 6 Kohlenstoffatome. Beispiele monocyclischer Radikale umfassen Cyclopropyl, Cyclobutyl, Cyclopentyl und Cyclohexyl.

Der Begriff "gegebenenfalls substituiertes Phenyl", wie hierin verwendet, steht für unsubstituiertes Phenyl oder beschreibt Phenyl, das 1, 2, 3, 4, oder 5, insbesondere 1, 2 oder 3 Substituenten trägt, die unter den Bedingungen der Reaktion inert sind. Beispiele für inerte Substituenten sind Halogen, insbesondere Fluor, Chlor oder Brom, CN, NO₂, C₁-C₆-Alkyl, C₁-C₆-Alkylthio, C₁-C₆-Alkylsulfonyl, C₁-C₄-Halogenalkyl, C₁-C₆-Alkoxy, C₃-C₆-Cycloalkyl, und C₁-C₄-Alkoxy-C₁-C₆-alkyl.

Der Begriff "gegebenenfalls substituiertes Phenyl-C₁-C₆-alkyl", wie hierin verwendet, beschreibt C₁-C₆-Alkyl, worin eines der Wasserstoffatome durch eine gegebenenfalls substituierte Phenylgruppe ersetzt ist. Beispiele sind Benzyl, 4-Methylbenzyl, Phenylethyl etc.

Der Begriff "N-gebundener 5- bis 8-gliedriger, gesättigter, gegebenenfalls substituierter Heterocyclus" steht für einen über ein Ringstickstoffatom gebundenen gesättigten Heterocyclus, der 5, 6, 7 oder 8 Ringatome aufweist, wobei neben dem Stickstoffatom die Ringatome auch weitere Heteroatome umfassen können, und der unsubstituiert ist oder 1, 2, 3, 4, oder 5, insbesondere 1, 2 oder 3 Substituenten trägt, die unter den Bedingungen der Reaktion inert sind. Beispiele für inerte Substituenten sind CN, C₁-C₆-Alkyl, C₁-C₆-Alkylthio, C₁-C₆-Alkylsulfonyl, C₁-C₄-Halogenalkyl, C₁-C₆-Alkoxy, C₃-C₆-Cycloalkyl, und C₁-C₄-Alkoxy-C₁-C₆-alkyl. Der Heterocyclus kann neben dem Stickstoffatom in Position 1 und den Ringkohlenstoffatomen noch 1 oder 2 weitere Heteroatome ausgewählt unter N, O und S als Ringatome aufweisen. Beispiele für N-gebundene 5- bis 8-gliedrige, gesättigte, gegebenenfalls substituierte Heterocyclen sind Pyrrolidin-1-yl, Piperdin-1-yl, Morpholin-4-yl, Piperazin-1-yl und N-Methylpiperazin-1-yl.

Die Erfindung betrifft die Herstellung von Pyrazolverbindungen der Formel I, worin R² für eine Gruppe COOR^{2a} steht, worin R^{2a} die zuvor genannten Bedeutungen aufweist und insbesondere für C₁-C₆-Alkyl oder C₁-C₄-Alkoxy-C₁-C₆-alkyl und speziell für C₁-C₄-Alkyl steht. Dementsprechend steht auch in den Formeln II und VI die Gruppe R² für eine Gruppe COOR^{2a}, worin R^{2a} die zuvor genannten Bedeutungen aufweist und insbesondere für C₁-C₆-Alkyl oder C₁-C₄-Alkoxy-C₁-C₆-alkyl und speziell für C₁-C₄-Alkyl steht.

Das erfindungsgemäße Verfahren eignet sich insbesondere zur Herstellung von Verbindungen der allgemeinen Formel I, worin X für eine Gruppe CX¹X²X³ steht, worin X¹, X² und X³ die zuvor genannten Bedeutungen haben, wobei wenigstens einer der Reste X¹ und X² von Wasserstoff verschieden ist. Insbesondere steht X¹ und X² für Fluor. X³ steht vorzugsweise für Wasserstoff, Fluor oder Chlor. Beispiele für bevorzugte Reste CX¹X²X³ sind Dichlormethyl, Chlorfluormethyl, Difluormethyl, Chlordifluormethyl und Trifluormethyl. Gemäß einer speziellen Ausführungsform steht X für eine Gruppe CHF₂.

Weiterhin hat es sich als vorteilhaft erwiesen, wenn R¹ in Formel I und dementsprechend in Formel III für Methyl steht.

Gemäß einer ersten Ausführungsform der Erfindung setzt man zur Herstellung der Pyrazolverbindungen der Formel I eine Verbindung der Formel II ein, worin Y für Sauerstoff steht. Derartige Verbindungen werden im Folgenden auch als Verbindungen IIa bezeichnet. Verbindungen der Formel IIa, worin R² für eine Gruppe COOR^{2a} steht, worin R^{2a} die zuvor genannten Bedeutungen aufweist und insbesondere für C₁-C₆-Alkyl oder C₁-C₄-Alkoxy-C₁-C₆-alkyl und speziell für C₁-C₄-Alkyl steht, werden im Folgenden auch als Verbindungen IIa.1 bezeichnet.

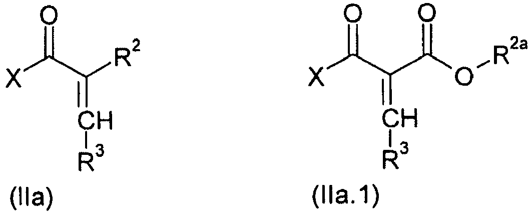

In den Formeln IIa und IIa.1 haben R², R^{2a}, R³ und X die zuvor genannten Bedeutungen.

Insbesondere steht X in den Formeln IIa und IIa.1 für eine Gruppe CX¹X²X³, worin X¹, X² und X³ die zuvor genannten Bedeutungen haben. Insbesondere ist wenigstens einer der Reste X¹ und X² von Wasserstoff verschieden. Insbesondere stehen X¹ und X² für Fluor. X³ steht vorzugsweise für Wasserstoff, Fluor oder Chlor. Beispiele für besonders bevorzugte Gruppe CX¹X²X³ sind Dichlormethyl, Trifluormethyl, Chlordifluormethyl, Fluorchlormethyl und Difluormethyl. Gemäß einer speziellen Ausführungsform steht X für eine Gruppe CHF₂.

Gemäß einer zweiten Ausführungsform der Erfindung setzt man zur Herstellung der Pyrazolverbindungen der Formel I eine Verbindung der Formel II ein, worin Y für eine Gruppe [NR^{y2}R^{y3}]⁺Z steht. Derartige Verbindungen werden im Folgenden auch als Verbindungen IIb bezeichnet. Verbindungen der Formel IIb, worin R² für eine Gruppe COOR^{2a} steht, worin R^{2a} die zuvor genannten Bedeutungen aufweist und insbesondere für C₁-C₆-Alkyl oder C₁-C₄-Alkoxy-C₁-C₆-alkyl steht, werden im Folgenden auch als Verbindungen IIb.1 bezeichnet.

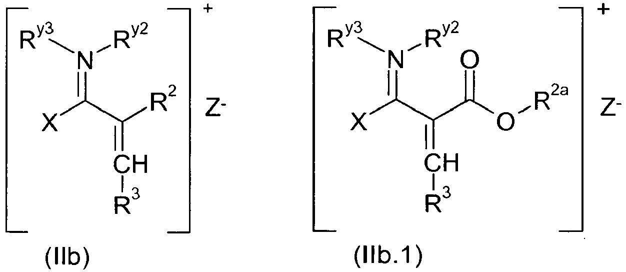

In den Formeln IIb und IIb.1 haben R², R^{2a}, R^{y2}, R^{y3}, Z, R³ und X die zuvor genannten Bedeutungen.

Insbesondere steht X in den Formeln IIb und IIb.1 für eine Gruppe CX¹X²X³, worin X¹, X² und X³ die zuvor genannten Bedeutungen haben. Insbesondere ist wenigstens einer der Reste X¹ und X² von Wasserstoff verschieden. Insbesondere steht X¹ und X² für Fluor. X³ steht vorzugsweise für Wasserstoff, Fluor oder Chlor. Beispiele für besonders bevorzugte Gruppe CX¹X²X³ sind Trifluormethyl, Chlordifluormethyl, Fluorchlormethyl und Difluormethyl. Insbesondere steht die Gruppe CX¹X²X³ in den Formeln IIb, IIb.1 und IIb.2 für CHCIF oder CHF₂.

R^{y2} und R^{y3} stehen insbesondere für C₁-C₄-Alkyl und speziell für Methyl.

Z- steht für ein Anion bzw. ein Anionenäquivalent, das vorzugsweise von einer Lewissäure wie MgF₂, BF₃, BCls, AICl₃, AlF₃, ZnCl₂, PF₅, SbF₅, BiCl₃, GaCl₃ SnCl₄, oder SiCl₄ abgeleitet ist, z.B. für Fluorid, [MgF_{3]}⁻, [BF_{4]}-, [BCl₃F]⁻, [AlF₄]⁻, [AlCl₃F]⁻, [ZnCl₂F]⁻, [PF6]⁻, [SbF₆]⁻, [BiCl₃F]⁻, [GaCl₃F]⁻, [SnCl₄F]⁻ oder [SiCl₄F]⁻.

Gemäß einer ersten Variante des erfindungsgemäßen Verfahrens steht R³ in den Formeln II, IIa und IIa.1, IIb und IIb.1 für eine Gruppe OR^{3a}. Hierbei hat R^{3a} die zuvor genannten Bedeutungen und steht insbesondere für C₁-C₄-Alkyl und speziell für Methyl oder Ethyl.

Gemäß einer zweiten Variante des erfindungsgemäßen Verfahrens steht R³ in den Formeln II, IIa und IIa.1, IIb und IIb.1 für eine Gruppe NR^{3b}R^{3c}. Hierbei haben R^{3b} und R^{3c} die zuvor genannten Bedeutungen und stehen insbesondere für C₁-C₄-Alkyl und speziell für Methyl oder Ethyl, oder R^{3b} und R^{3c} bilden zusammen mit dem Stickstoffatom, an das diese gebunden sind für einen N-gebundenen 5- bis 8-gliedrigen, gesättigten Heterocyclus stehen, der neben dem Stickstoffatom noch 1 oder 2 weitere Heteroatome ausgewählt unter N, O und S als Ringatome aufweisen kann und der gegebenenfalls 1 oder 2 C₁-C₄-Alkylgruppen tragen kann. Beispiele für die letztgenannte cyclische Gruppe NR^{3b}R^{3c} sind Pyrrolidin-1-yl, Morpholin-4-yl, Piperidin-1-yl und 4-Methylpiperazin-1-yl.

Die Art des in der Umsetzung eingesetzten Hydrazons der Formel III ist grundsätzlich von untergeordneter Bedeutung. Grundsätzlich sind solche Hydrazone der Formel III (und dementsprechend auch Verbindungen der Formel VI) bevorzugt, worin
- R⁴: für Wasserstoff oder C₁-C₆-Alkyl steht und
- R⁵: für C₁-C₆-Alkyl, C₃-C₆-Cycloalkyl oder gegebenenfalls substituiertes Phenyl steht, oder
- R⁴ und R⁵: gemeinsam mit dem Kohlenstoffatom, an das sie gebunden sind, für einen 5- bis 10-gliedrigen gesättigten Carbocyclus stehen können, der gegebenenfalls ein oder mehrfach, z.B. 1-, 2-, 3- oder 4-fach, durch C₁-C₄-Alkylgruppen substituiert ist und/oder einen kondensierten Phenylring aufweist.

Gemäß einer besonders bevorzugten Ausgestaltung des erfindungsgemäßen Verfahrens setzt man ein Hydrazon der Formel III ein, worin
- R⁴: für Wasserstoff oder C₁-C₄-Alkyl, insbesondere für Wasserstoff, steht und
- R⁵: für gegebenenfalls substituiertes Phenyl steht.

Gemäß einer anderen besonders bevorzugten Ausgestaltung des erfindungsgemäßen Verfahrens setzt man ein Hydrazon der Formel III ein, worin R⁴ und R⁵ für C₁-C₄-Alkyl stehen oder gemeinsam mit dem Kohlenstoffatom, an das sie gebunden sind einen 5 bis 8 gliedrigen, gesättigten Carbocyclus bilden, der gegebenenfalls in der oben beschriebenen Weise substituiert ist.

Der Ausdruck "gegebenenfalls substituiertes Phenyl" hat hierbei die zuvor genannten Bedeutungen und steht insbesondere für unsubstituiertes Phenyl oder für Phenyl, das 1, 2 oder 3 Substituenten aufweist, ausgewählt unter Halogen, insbesondere Fluor, Chlor oder Brom, Nitro, Cyano, C₁-C₄-Alkyl, insbesondere Methyl oder Ethyl, und C₁-C₄-Alkoxy, insbesondere Methoxy oder Ethoxy, z.B. wie in 2-, 3- oder 4-Fluorphenyl, 2-, 3- oder 4-Chlorphenyl, 4-Bromphenyl, 2-, 3- oder 4-Methylphenyl, 2-, 3- oder 4-Methoxyphenyl, 4-Cyanophenyl, 4-Nitrophenyl.

Im Hinblick auf R⁵ hat der Ausdruck "gegebenenfalls substituiertes Phenyl" die zuvor genannten Bedeutungen und steht besonders bevorzugt für unsubstituiertes Phenyl oder für Phenyl, das 1 oder 2 Substituenten aufweist, ausgewählt unter Halogen, insbesondere Chlor, C₁-C₄-Alkyl, insbesondere Methyl oder Ethyl, und C₁-C₄-Alkoxy, insbesondere Methoxy oder Ethoxy z.B. wie in 2-, 3- oder 4-Chlorphenyl, 2-, 3- oder 4-Methylphenyl, 2-, 3- oder 4-Methoxyphenyl.

Gemäß einer ganz besonders bevorzugten Ausgestaltung des erfindungsgemäßen Verfahrens setzt man ein Hydrazon der Formel III ein, worin
- R⁴: für Wasserstoff steht und
- R⁵: für gegebenenfalls substituiertes Phenyl, insbesondere für unsubstituiertes Phenyl oder für Phenyl, das 1 oder 2 Substituenten aufweist, steht, wobei die Substituenten wie zuvor genannt sind und vorzugsweise ausgewählt sind unter Halogen, insbesondere Chlor, C₁-C₄-Alkyl, insbesondere Methyl oder Ethyl, und C₁-C₄-Alkoxy, insbesondere Methoxy oder Ethoxy.

Gemäß einer ganz besonders bevorzugten Ausgestaltung des erfindungsgemäßen Verfahrens setzt man ein Hydrazon der Formel III ein, worin
- R⁴ und R⁵: gemeinsam mit dem Kohlenstoffatom, an das sie gebunden sind, für einen 5- bis 10-gliedrigen, insbesondere 5 bis 8-gliedrigen gesättigten Carbocyclus stehen, der gegebenenfalls ein oder mehrfach, z.B. 1-, 2-, 3- oder 4-fach, durch C₁-C₄-Alkylgruppen substituiert ist.

Die Umsetzung der Verbindungen der Formel II mit dem Hydrazon der Formel III in Schritt i) des erfindungsgemäßen Verfahrens erfolgt üblicherweise bei Temperaturen im Bereich von 0 bis 180°C, insbesondere im Bereich von 10 bis 150°C.

Zur Umsetzung werden die Verbindungen II und III vorzugsweise in einem der Stöchiometrie der Reaktion entsprechenden Verhältnis eingesetzt, wobei von der Stöchiometrie auch abgewichen werden kann. Typischerweise liegt das Molverhältnis von Verbindung II zu Verbindung III im Bereich von 1,5:1 bis 1:1,5, häufig im Bereich von 1,2:1 bis 1:1,2 und insbesondere im Bereich von 1,1:1 bis 1:1,1.

Typischerweise erfolgt die Umsetzung in Schritt i) in einem inerten organischen Lösungsmittel. Beispiele für inerte organische Lösungsmittel sind insbesondere aprotische organische Lösungsmittel wie aromatische Kohlenwasserstoffe und Halogenkohlenwasserstoffe, z.B. Benzol, Toluol, Xylole, Cumol, Chlorbenzol und tert.-Butylbenzol, cyclische oder acyclische Ether wie Diethylether, Diisopropylether, tert.-Butylmethylether (MTBE), tert.-Butylethylether, Tetrahydrofuran (THF) oder Dioxan, Nitrile wie Acetonitril und Propionitril, aliphatische Halogenkohlenwasserstoffe wie Dichlormethan, Dichlorethan, Trichlormethan und deren Gemische. Vorzugsweise arbeitet man in Schritt i) im Wesentlichen wasserfrei, d.h. der Wassergehalt in der Lösung liegt unterhalb 1 %, insbesondere unterhalb 0,1 %, bezogen auf das Gesamtgewicht des Lösungsmittels.

Zur Umsetzung der Verbindungen der Formel II mit Hydrazonen der Formel III wird man in der Regel so vorgehen, dass man die Verbindung der Formel II, vorzugsweise in Form einer Lösung in einem der vorgenannten inerten organischen Lösungsmittel, mit dem Hydrazon III, das ebenfalls vorzugsweise als Lösung in einem der vorgenannten inerten organischen Lösungsmittel eingesetzt wird, zusammengibt. Hierbei kann man das Hydrazon III als Lösung in einem organischen Lösungsmittel vorlegen und die Verbindung II, vorzugsweise als Lösung zugeben. Alternativ kann man die Verbindung II als Lösung in einem organischen Lösungsmittel vorlegen und das Hydrazon, vorzugsweise als Lösung zugeben. Das Zusammengeben des Hydrazons III und der Verbindung II kann in den oben genannten Temperaturbereichen erfolgen. Häufig wird man so vorgehen, dass das Zusammengeben der Verbindungen II und III bei Temperaturen im Bereich von 0 bis 50°C, insbesondere 10 bis 50°C erfolgt und anschließend die Reaktionsmischung auf die gewünschte Temperatur erwärmt wird. Die Umsetzungsdauer liegt typischerweise im Bereich von 1 h bis 15 h.

Auf diese Weise erhält man die Verbindung der Formel VI, die aus dem Reaktionsgemisch isoliert werden kann. Alternativ kann man das Reaktionsgemisch auch ohne Isolierung der Verbindung VI der Umsetzung in Schritt ii) des erfindungsgemäßen Verfahrens zuführen. Eine Fahrweise ohne Isolierung der Zwischenverbindung VI ist von Vorteil, da auf diese Weise Ausbeuteverluste wie sie z.B. bei der Feststoffabtrennung der Zwischenverbindung durch Filtration auftreten (z.B. Verluste in der Mutterlauge) verringert oder vermieden werden. In diesen Fällen kann man gegebenenfalls eine Teilmenge des in Schritt i) eingesetzten organischen Lösungsmittels entfernen und gegebenenfalls durch ein anderes Lösungsmittel ersetzen. Eine Fahrweise ohne Isolierung der Zwischenverbindung VI ist insbesondere auch von Vorteil, wenn in der eingesetzten Verbindung II die Gruppe Y für [NR^{y2}R^{y3}]⁺Z⁻ steht.

Erfindungsgemäß erfolgt die Umsetzung in Gegenwart einer Säure, insbesondere einer Brönstedt-Säure. Bevorzugte Säuren weisen einen pKs-Wert von nicht mehr als 4, insbesondere nicht mehr als 3 oder nicht mehr als 2 auf (in verdünnter (z.B. 0.01 M) wässriger Lösung bei 25°C). Bevorzugte Säuren sind Halogenwasserstoffsäuren wie HF, HCl und HBr, insbesondere in Form ihrer wässrigen Lösungen, Schwefelsäure, Phosphorsäure, HBF₄, und organische Sulfonsäuren, z.B. aromatische Sulfonsäuren der Formel Ar-SO₃H, worin Ar für gegebenenfalls substituiertes Phenyl steht, wie Benzolsulfonsäure und p-Toluolsulfonsäure, sowie aliphatische Sulfonsäuren wie Methansulfonsäure, Ethansulfonsäure und Trifluormethansulfonsäure. Ebenfalls geeignet sind aliphatische und aromatische Carbonsäuren wie Ameisensäure, Chloressigsäure, Dichloressigsäure, Trichloressigsäure, Trifluoressigsäure, Salicylsäure und 2-Chlorbenzoesäure. Geeignet sind selbstverständlich auch Gemische der vorgenannten Säuren.

Für die Umsetzung in Schritt ii) reichen in der Regel katalytische Säuremengen aus. Die Säure kann jedoch auch in stöchiometrischer oder überstöchiometrischer Menge eingesetzt werden. In der Regel setzt man die Säure in einer Menge von 0,01 bis 10 Mol und insbesondere in einer Menge von 0,02 bis 5 Mol pro Mol Verbindung VI, bzw. im Falle einer in-situ Herstellung der Verbindung VI, in einer Menge von 0,01 bis 10 Mol und insbesondere in einer Menge von 0,02 bis 2 Mol pro Mol Verbindung II ein.

Die Umsetzung in Schritt ii) des erfindungsgemäßen Verfahrens erfolgt erfindungsgemäß in Gegenwart von Wasser. Man nimmt an, dass das Wasser zur Spaltung der Hydrazongruppe in der in Schritt I gebildeten Verbindung der Formel VI unter Bildung der Verbindung VIa (dargestellt für Y = O) führt, welche dann zum Pyrazol cyclisiert. Das erfindungsgemäße Verfahren lässt sich für Y = O anhand des folgenden Schemas 1 darstellen:

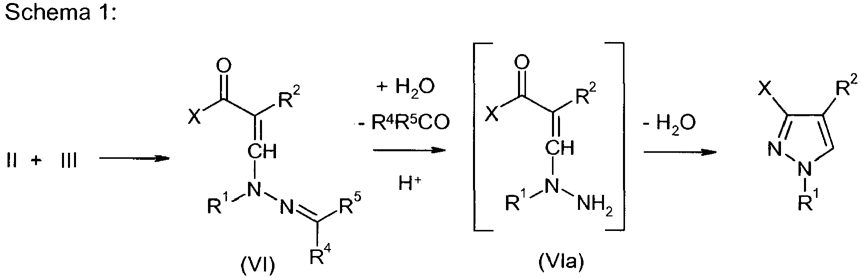

Wie aus dem Schema ersichtlich, reicht im Falle von Y = O bereits die Anwesenheit katalytischer Mengen an Wasser zur Umsetzung aus, da bei der Reaktion Wasser gebildet wird. Wasser kann auch in stöchiometrischer oder überstöchiometrischer Menge eingesetzt werden. In der Regel setzt man Wasser in einer Menge von 0,001 bis 50 Mol und insbesondere in einer Menge von 0,01 bis 20 Mol pro Mol Verbindung VI, bzw. im Falle einer in-situ Herstellung der Verbindung VI, in einer Menge von 0,001 bis 50 Mol und insbesondere in einer Menge von 0,01 bis 20 Mol pro Mol Verbindung II ein. Man nimmt an, dass die Umsetzung von Verbindung II, worin Y für NR^{y1} oder [NR^{y2}R^{y3}]⁺Z⁻ steht, mit dem Hydrazon III, und die anschließende Cyclisierung zur Pyrazolverbindung I in analoger Weise verläuft, wobei jedoch im Unterschied zu der Variante mit Y = O wenigstens stöchiometrische Mengen an Wasser für einen vollständigen Umsatz bei der Cyclisierung benötigt werden. Dementsprechend setzt man in diesem Fall das Wasser typischerweise in einer Menge von 1 bis 50 Mol und insbesondere in einer Menge von 1,1 bis 20 Mol pro Mol Verbindung VI, bzw. im Falle einer in-situ Herstellung der Verbindung VI, in einer Menge von 1 bis 50 Mol und insbesondere in einer Menge von 1,1 bis 20 Mol pro Mol Verbindung II ein.

Typischerweise erfolgt die Umsetzung in Schritt ii) in Gegenwart eines organischen Lösungsmittels oder Lösungsmittelgemischs. Geeignete organische Lösungsmittel für die Umsetzung in Schritt ii) sind protisch polare Lösungsmittel, z.B. aliphatische Alkohole mit vorzugsweise 1 bis 4 Kohlenstoffatomen, wie Methanol, Ethanol, n-Propanol, Isopropanol, n-Butanol, Isobutanol oder tert.-Butanol, oder Carbonsäuren, wie Essigsäure, aromatische Kohlenwasserstoffe, wie Benzol, Toluol, Xylole, Cumol, Chlorbenzol, Nitrobenzol oder tert.-Butylbenzol, aprotische polare Lösungsmittel, z.B. cyclische oder acyclische Ether wie Diethylether, Diisopropylether, tert.-Butylmethylether (MTBE), tert.-Butylethylether, Tetrahydrofuran (THF) oder Dioxan, cyclische oder acyclische Amide, wie Dimethylformamid, Dimethylacetamid, N-Methylpyrrolidon oder Tetramethylharnstoff, oder aliphatische Nitrile wie Acetonitril oder Propionitril, sowie Mischungen der zuvor genannten Lösungsmittel.

Zur Umsetzung in Schritt ii) geht man in der Regel so vor, dass man die in Schritt i) des erfindungsgemäßen Verfahrens hergestellte Verbindung der Formel VI bzw. das in Schritt i) erhaltene Reaktionsgemisch, gegebenenfalls nach einem teilweisen oder vollständigen Austausch des in Schritt i) eingesetzten Lösungsmittel, in einem geeigneten organischen Lösungsmittel vorlegt und hierzu Säure und Wasser gibt. Es ist möglich, das für die Umsetzung benötigte Wasser über das organische Lösungsmittel einzubringen. Es ist ebenso möglich, das für die Umsetzung benötigte Wasser über Säure einzubringen, z.B. in Form einer wässrigen Lösung der Säure oder in Form eines Hydrats der Säure.

Die Umsetzung in Schritt ii) des erfindungsgemäßen Verfahrens erfolgt üblicherweise bei Temperaturen im Bereich von 0 bis 150°C, insbesondere im Bereich von 20 bis 110°C. Die Umsetzungsdauer liegt typischerweise im Bereich von 0,1 h bis 15 h.

In Schritt ii) fällt die gewünschte 1,3-Pyrazolverbindung I in hoher Ausbeute und hoher Selektivität, d.h. mit sehr geringen oder nicht nachweisbaren Anteil an unerwünschtem 1,5-Isomer I' an. So beträgt das Molverhältnis von 1,3-Isomer der Formel I zu 1,5-Isomer der Formel I' in der Regel wenigstens 20:1, häufig wenigstens 50:1, insbesondere wenigstens 80 : 1 und speziell wenigstens 100 : 1.

Die gewünschte 1,3-Pyrazolverbindung I kann aus dem Reaktionsgemisch nach üblichen Methoden durch Fällung, Kristallisation oder Destillation isoliert werden oder in Form des Reaktionsgemischs zu Folgeprodukten weiterverarbeitet werden.

Die im erfindungsgemäßen Verfahren eingesetzten Verbindungen der Formel II sind bekannt, z.B. aus dem eingangs zitierten Stand der Technik oder können in Analogie zu den dort beschriebenen Methoden hergestellt werden.

Verbindungen der Formel II, worin Y für Sauerstoff steht und R³ für eine Gruppe OR^{3a} steht, sind beispielsweise aus US 5,498,624, JACS, 73, 3684, WO 92/12970, Chem. Ber. 1982, 115, 2766, Journal of Medicinal Chemistry, 2000, Vol. 43, No. 21 und den älteren Anmeldungen PCT/EP2007/061833 und EP 07109463.5 bekannt oder können in Analogie zu den dort beschriebenen Verfahren hergestellt werden, z.B. durch Umsetzung von Acrylverbindungen der Formel IX (R² = CO₂R^{2a}) mit Acylhalogeniden (Q = Halogen) oder Acylanhydriden (Q = OC(O)X) der Formel X gemäß dem folgenden Schema 2a oder durch Umsetzung von ß-Ketoestern der Formel XI (R² = CO₂R^{2a}) mit ortho-Ameisensäureestern der Formel XII gemäß dem folgenden Schema 2b.

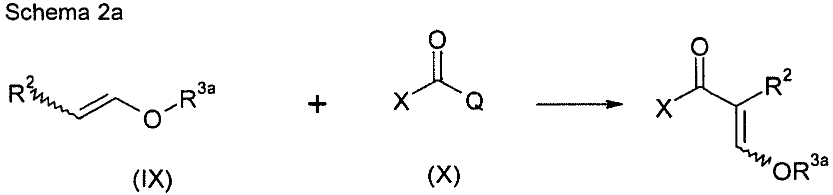

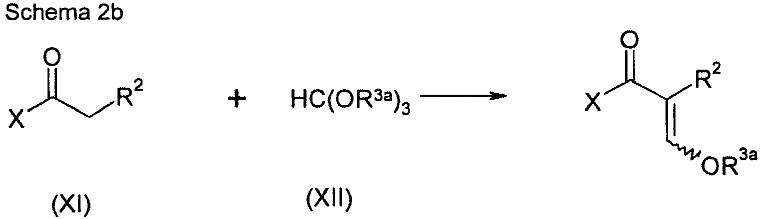

In den Schemata 2a und 2b haben die Variablen R², R^{3a} und X die zuvor genannten Bedeutungen. Q steht insbesondere für Fluor, Chlor oder einen Rest OC(O)X, worin X eine der zuvor genannten Bedeutungen aufweist.

Verbindungen der Formel II, worin Y für Sauerstoff steht und R³ für eine Gruppe NR^{3b}R^{3c} steht, sind beispielsweise aus WO 03/051820, WO 2005/042468 und den älteren Anmeldungen PCT/EP2007/064390, EP 08155612.8 und EP 08155611.0 bekannt oder können in Analogie zu den dort beschriebenen Verfahren hergestellt werden. Verbindungen der Formel II mit R² = CO₂R^{2a} kann man beispielsweise durch Umsetzung entsprechender 3-Aminoacrylverbindungen XIII mit den in Schema 2 beschriebenen Acylverbindungen der Formel X gemäß der in Schema 3 gezeigten Umsetzung herstellen.

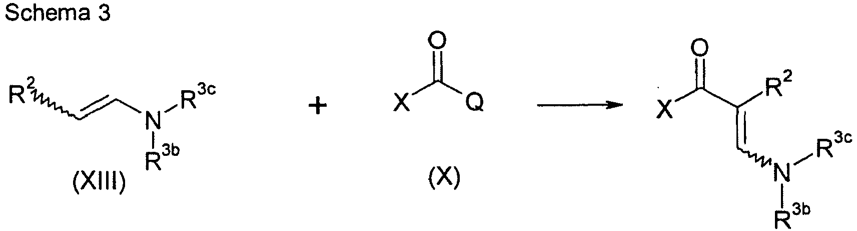

Verbindungen der Formel II, worin Y für eine Gruppe [NR^{y1}R^{y2}]Z⁻ steht (Verbindungen IIb), können beispielsweise nach den in WO 2008/022777 und der älteren Anmeldung EP 07110397.2 beschriebenen Verfahren hergestellt werden. Danach erfolgt die Herstellung von II, worin Y für eine Gruppe [NR^{y1}R^{y2}]Z⁻ steht, typischerweise durch Umsetzung von α,α-Difluoraminen der Formel XIV mit einer olefinischen Verbindung der Formel XV in Gegenwart einer Lewissäure wie MgF₂, BF₃, BCl₃, AlCl₃, AlF₃, ZnCl₂, PF₅, SbF₅, BiCl₃, GaCl₃, SnCl₄, oder SiCl₄ nach dem in Schema 4 dargestellten Verfahren.

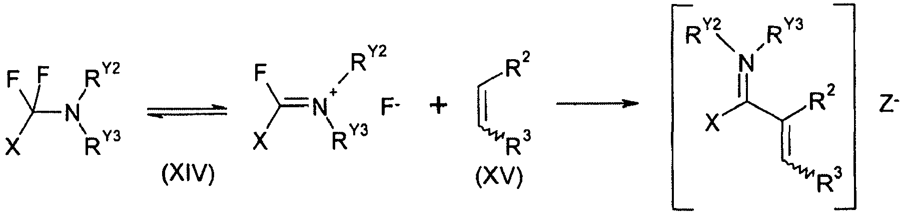

Hierbei hat es sich bewährt, die durch Umsetzung von XIV mit XV erhaltenen Iminiumverbindung IIb nicht zu isolieren sondern die erhaltene Reaktionsmischung, ggf. nach Entfernen einer Teilmenge des Lösungsmittels, in der Umsetzung mit dem Hydrazon der Formel III einzusetzen. Wegen Details zur Herstellung der Verbindungen IIb wird insbesondere auf die Offenbarung der WO 2008/022777 und der älteren Anmeldung WO 2008/152138 (vormals EP 07110397.2) verwiesen, auf die hiermit Bezug genommen wird.

Die im erfindungsgemäßen Verfahren eingesetzten Hydrazonverbindungen der Formel III sind bekannt oder können in an sich bekannter Weise durch Umsetzung einer Carbonylverbindung der Formel IV mit einer substituierten Hydrazinverbindung der Formel V hergestellt werden.

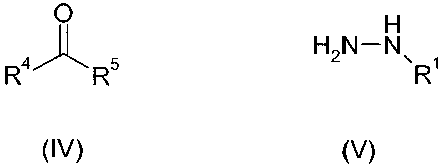

In den Formeln IV und V haben R¹, R⁴ und R⁵ die für Formel III bzw. VI angegebenen Bedeutungen. Die Umsetzung der Verbindungen IV und V zum Hydrazon III kann in an sich bekannter Weise erfolgen.

Die Umsetzung der Carbonylverbindung IV mit der Hydrazinverbindung V erfolgt üblicherweise bei Temperaturen im Bereich von 10 bis 180°C, insbesondere im Bereich von 20 bis 150°C.

Zur Umsetzung werden die Verbindungen IV und V vorzugsweise in einem der Stöchiometrie der Reaktion entsprechenden Verhältnis eingesetzt, wobei von der Stöchiometrie auch abgewichen werden kann. Typischerweise liegt das Molverhältnis von Verbindung IV zu Verbindung V im Bereich von 1,5:1 bis 1:1,5, häufig im Bereich von 1,2:1 zu 1:1,2 und insbesondere im Bereich von 1,1:1 bis 1:1,1.

Typischerweise erfolgt die Umsetzung von IV mit V in einem inerten organischen Lösungsmittel. Beispiele für inerte organische Lösungsmittel sind insbesondere aprotische organische Lösungsmittel wie aromatische Kohlenwasserstoffe und Halogenkohlenwasserstoffe, z.B. Benzol, Toluol, Xylole, Cumol, Chlorbenzol und tert.-Butylbenzol, cyclische oder acyclische Ether wie Diethylether, Diisopropylether, tert.-Butylmethylether (MTBE), tert.-Butylethylether, Tetrahydrofuran (THF) oder Dioxan, Nitrile wie Acetonitril und Propionitril, aliphatische Halogenkohlenwasserstoffe wie Dichlormethan, Dichlorethan, Trichlormethan und deren Gemische.

Zur Umsetzung der Verbindungen der Formel IV mit der Hydrazinverbindung der Formel V wird man in der Regel so vorgehen, dass man die Verbindung der Formel IV, vorzugsweise in Form einer Lösung in einem der vorgenannten inerten organischen Lösungsmittel, mit der Hydrazinverbindung V, vorzugsweise als Lösung in Wasser, zusammengibt. Das Zusammengeben der Verbindungen IV und V kann in den oben genannten Temperaturbereichen erfolgen. Häufig wird man so vorgehen, dass das Zusammengeben der Verbindungen IV und V bei Temperaturen im Bereich von 0 bis 50°C, insbesondere 10 bis 50°C erfolgt und anschließend die Reaktionsmischung auf die gewünschte Temperatur erwärmt wird. Die Umsetzungsdauer liegt typischerweise im Bereich von 0,5 h bis 8 h.

In der Regel hat es sich als vorteilhaft erwiesen, das bei der Reaktion gebildete Wasser oder das durch Verwendung einer wässrigen Lösung des Hydrazins V eingebrachte Wasser zu entfernen, z.B. durch Destillation, Wasserauskreisung, mittels eines Schleppmittels, durch Phasentrennung, sonstige Trocknung oder ein Kombination dieser Maßnahmen.

Das Hydrazon kann aus dem durch Umsetzung von IV mit V erhaltenen Reaktionsgemisch isoliert werden oder als Reaktionsgemisch in der Folgestufe, d.h. in Schritt I des erfindungsgemäßen Verfahrens eingesetzt werden.

Ein weiterer Gegenstand der vorliegenden Erfindung betrifft ein Verfahren zur Herstellung einer Verbindung der allgemeinen Formel la 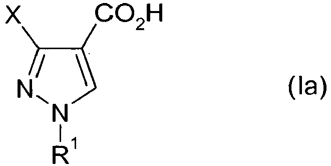 worin X und R¹ die zuvor angegebenen Bedeutungen aufweisen, umfassend die folgenden Schritte
a) Bereitstellung einer Pyrazolverbindung der Formel I nach einem Verfahren gemäß dem hier beschriebenen Verfahren,
b) Umwandlung der Verbindung I in eine 1,3-substituierte Pyrazolcarbonsäure der Formel la.

Die Umwandlung erfolgt typischerweise durch Hydrolyse. Dementsprechend betrifft eine bevorzugte Ausführungsform der Erfindung ein Verfahren mit den folgenden Schritten:
a) die Bereitstellung einer Verbindung der Formel I nach dem erfindungsgemäßen Verfahren wie beschrieben und
b) eine Hydrolyse der Verbindung I unter Bildung einer 1,3-substituierten Pyrazol-4-ylcarbonsäure der Formel la.

Die Hydrolyse kann unter Säurekatalyse oder basisch oder in sonstiger Weise durchgeführt werden. Die Verbindung I kann als solche, d. h. nach Isolierung eingesetzt werden. Es ist jedoch auch möglich das in Schritt a) erhaltene Reaktionsgemisch, gegebenenfalls nach Abtrennung flüchtiger Bestandteile wie Lösungsmittel, ohne weitere Reinigung zur Hydrolyse einzusetzen.

Zur basischen Hydrolyse der Verbindung I wird man typischerweise die Verbindung der Formel I mit einem Alkalimetallhydroxid wie Natriumhydroxid, Kaliumhydroxid oder Lithiumhydroxid, bevorzugt mit einer wässrigen Alkalimetallhydroxid-Lösung, speziell einer wässrigen NaOH-Lösung oder einer wässrigen KOH-Lösung, bis zur vollständigen Hydrolyse des Esters behandeln, vorzugsweise unter Erwärmen.

Bei der basischen Hydrolyse liegt das Molverhältnis von Verbindung der Formel I zu Base typischerweise im Bereich von 1,2 : 1 bis 1 : 10 und ist insbesondere etwa äquimolar (d. h. es liegt im Bereich von 1,1 : 1 bis 1 : 1,5), jedoch kann auch ein größerer Basenüberschuss, z. B. bis zu 5 Mol je Mol Verbindung I, von Vorteil sein.

Üblicherweise erfolgt die basische Hydrolyse in einem Verdünnungs- bzw. Lösungsmittel. Geeignete Verdünnungs- bzw. Lösungsmittel sind neben Wasser auch organische Lösungsmittel, die gegenüber Alkali stabil sind, sowie deren Mischungen mit Wasser. Beispiele für Alkali-stabile organische Lösungsmittel sind insbesondere die vorgenannten C₁-C₄-Alkanole sowie die vorgenannten acyclischen und die cyclischen Ether. Vorzugsweise führt man die Hydrolyse in wässriger Phase, d. h. in Wasser oder einer Mischung aus Wasser mit einem der vorgenannten organischen Lösungsmittel durch, wobei der Gehalt an organischem Lösungsmittel in der wässrigen Phase in der Regel 30 Vol.-%, bezogen auf die Gesamtmenge an Wasser und organischem Lösungsmittel typischerweise nicht überschreitet.

Vorzugsweise führt man die basische Hydrolyse bei Temperaturen von 20 bis 100 °C durch. In der Regel ist die obere Temperaturgrenze der Siedepunkt des verwendeten Lösungsmittels bei druckloser Reaktionsführung. Vorzugsweise wird man eine Reaktionstemperatur von 100 °C und insbesondere 90 °C nicht überschreiten. In einer bevorzugten Ausführungsform führt man jedoch die Basische Hydrolyse bei einer Temperatur unterhalb des Siedepunkts der Alkoholkomponente durch, z.B. bei Temperaturen im Bereich von 40 bis < 80°C, insbesondere im Bereich von 50 bis 75°C, insbesondere wenn von einer Verbindung der allgemeinen Formel I ausgeht, worin R¹ für Methyl oder Ethyl steht. Höhere Temperaturen sind jedoch ebenfalls möglich. So arbeitet man in einer anderen Ausführungsform der basischen Hydrolyse bei einer Temperatur oberhalb des Siedepunkts der Alkoholkomponente des Esters. Beispielsweise wird man dann die Hydrolyse bevorzugt bei einer Temperatur von wenigstens 80 °C, z. B. im Bereich von 80 bis 100 °C durchführen, z.B. wenn von einer Verbindung der allgemeinen Formel I ausgeht, worin R¹ für Ethyl steht. Die Reaktionsdauer ist hierbei von der Reaktionstemperatur, der Konzentration und der Stabilität der jeweiligen Esterbindung abhängig. Im Allgemeinen werden die Reaktionsbedingungen so gewählt, dass die Reaktionszeit im Bereich von 1 bis 12 h, insbesondere im Bereich von 2 bis 8 h, liegt.

In einer besonders bevorzugten Ausführungsform der Erfindung zur Herstellung einer Verbindung der allgemeinen Formel la wird die im Teilschritt a) erhaltene Pyrazolverbindung I in dem Fall, dass R² für CO₂R^{2a} steht, ohne Zwischenisolierung, vorteilhaft zusammen mit dem organischen Lösungsmittel, mit der wässrigen Alkalimetallhydroxid-Lösung umgesetzt. Das dabei gebildete Alkalimetallsalz der Pyrazolcarbonsäure la fällt dabei als wässrige Phase neben der organischen Phase an, die durch Phasentrennung abgetrennt werden kann. Auf diese Weise kann die bei der Umsetzung der Verbindungen II und III im Teilschritt ii) wieder freigesetzte Carbonylverbindung IV (R⁴R⁵C=O), insbesondere wenn R⁴ für gegebenenfalls substituiertes Phenyl steht, mit der organischen Phase abgetrennt werden. Eine Rückführung der Carbonylverbindung IV in den Reaktionsprozess zur Hydrazonbildung (gegebenenfalls nach vorheriger weiterer Aufarbeitung z. B. durch Destillation) ist somit möglich. Auch ein Recycling des verwendeten organischen Lösungsmittels kann dabei vorgenommen werden. Die bei der Phasentrennung anfallende wässrige Phase enthält das Alkalimetallsalz der 1,3-substituierten Säure la in der Regel in gelöster Form und. Das Salz kann dann wie oben beschrieben durch Sauerstellen der Lösung in die freie Säure la überführt werden. In der Regel fällt die Säure la dabei als Feststoff aus und kann durch Filtration isoliert und ggf. getrocknet werden. Bei dieser Verfahrensweise fällt die 1,3-substituierte Pyrazolcarbonsäure in hoher Reinheit und mit sehr guter Ausbeute an. Die Ausbeute, bezogen auf die eingesetzte Verbindung II beträgt in der Regel wenigstens 80 % und insbesondere wenigstens 85 %.

Die saure Hydrolyse der Verbindung I kann in Analogie zu bekannten sauren Esterhydrolysen durchgeführt werden, d. h. in Gegenwart katalytischer oder stöchiometrischer Mengen einer Säure und Wasser (siehe z. B. J. March, Advanced Organic Chemistry, 2nd Ed., 334-338, McGraw-HiII, 1977 und dort zitierte Literatur). Häufig wird man die Umsetzung in einer Mischung aus Wasser und einem aprotischen organischen Lösungsmittel, beispielsweise einem Ether wie zuvor genannt, durchführen. Beispiele für Säuren sind Halogenwasserstoffsäuren, Schwefelsäure, organische Sulfonsäuren wie p-Toluolsulfonsäure, Methansulfonsäure, Phosphorsäure sowie saure lonentauscherharze und dergleichen.

Geeignete Hydrolysekatalysatoren sind weiterhin Alkalimetalliodide, wie Lithiumiodid, Trimethyliodsilan oder Mischungen von Trimethylchlorsilan mit Alkalimetalliodiden wie Lithium-, Natrium- oder Kaliumiodid.

Die Isolierung der Säure la erfolgt dann durch übliche Trennverfahren, wie beispielsweise Fällung durch Einstellen des pH-Werts oder Extraktion.

Die Pyrazolverbindungen der Formel I, insbesondere die Pyrazolcarbonsäuren der Formel Ia, stellen wertvolle Zwischenprodukte bei der Herstellung von Wirkstoffen, die einen 1,3-substituierten Pyrazolrest aufweisen, insbesondere bei der Herstellung der fungiziden Wirkstoffe der im Folgenden beschriebenen Formel VII: 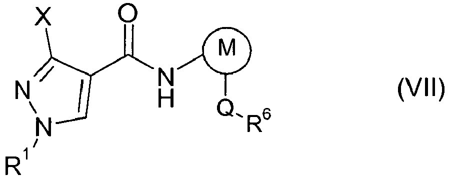 worin R¹ und X eine der in Anspruch 1 angegebenen Bedeutungen aufweisen;
- M: für Thienyl oder Phenyl, das einen Halogensubstituenten tragen kann, steht;
- Q: für eine direkte Bindung, Cyclopropylen, ein annellierter Bicyclo[2.2.1]heptan- oder Bicyclo[2.2.1]hepten-Ring steht; und
- R⁶: für Wasserstoff, Halogen, C₁-C₄-Alkyl, C₁-C₄-Halogenalkoxy, ein- bis dreifach substituiertes Phenyl, wobei die Substituenten unabhängig voneinander ausgewählt sind aus Halogen und Trifluormethylthio, oder Cyclopropyl steht.

Dementsprechend betrifft die vorliegende Erfindung auch ein Verfahren zur Herstellung einer Verbindung der Formel VII, umfassend die folgenden Schritte:
a) Bereitstellung einer Pyrazolverbindung der Formel I nach dem erfindungsgemäßen Verfahren;
b) Umwandlung der Verbindung I in eine 1,3-substituierte Pyrazolcarbonsäure der Formel la, 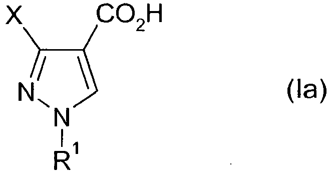 worin X und R¹ die zuvor angegebenen Bedeutungen aufweisen;
c) gegebenenfalls Überführung der Verbindung la in ihr Säurehalogenid; und
d) Umsetzung der Verbindung der Formel la oder ihres Säurehalogenids mit einer Aminverbindung der Formel VIII, 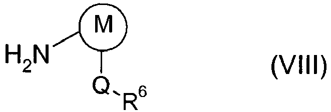
worin M, Q und R⁶ die für Formel VII angegebenen Bedeutungen aufweisen.

Geeignete Methoden zur Herstellung von Carbonsäuren und Umsetzung von Carbonsäuren oder Carbonsäurehalogeniden mit aromatischen Aminen sind dem Fachmann bekannt, z. B. aus dem eingangs zitierten Stand der Technik (siehe US 5,498,624, EP 545099 A1, DE 19531813 A1, EP 589301 A1, DE 19840322 A1, WO 92/12970, WO 03/066610, WO 2006/024389, WO 2007/003603, WO 2007/006806) sowie aus J. March, Advanced Organic Chemistry, 3rd ed. J. Wiley and Sons, New York 1985, S. 370-386 und dort zitierte Literatur sowie Organikum, 21. Auflage Wiley-VCH, Weinheim 2001, S. 481-484 und dort zitierte Literatur, und können auf die erfindungsgemäße Herstellung der Verbindungen VII durch Umsetzung der Pyrazolcarbonsäure la oder ihres Säurehalogenids mit der Anilinverbindung VII in analoger Weise übertragen werden.

Häufig wird man so vorgehen, dass man die Pyrazolcarbonsäure der Formel la zunächst in ihr Säurehalogenid, z. B. ihr Säurechlorid überführt und anschließend das Säurehalogenid mit der Aminverbindung der Formel VII umsetzt. Die Überführung der Pyrazolcarbonsäure in ihr Säurechlorid gelingt in Analogie zu Standardverfahren der organischen Chemie, beispielsweise durch Umsetzung mit Thionylchlorid. Die Anschließende Umsetzung des Säurehalogenids mit der Aminverbindung VIII erfolgt typischerweise in Gegenwart einer Hilfsbase, z.B. ein tertiäres Amin. Alternativ kann man die Pyrazolcarbonsäure der Formel la auch direkt mit der Aminverbindung VIII, vorzugsweise in Gegenwart eine Dehydratisierungsmittels wie 1,1'-Carbonyldiimidazol, Bis(2-oxo-3-oxazolidinyl)phosphorylchlorid, N,N'-Dicyclohexyl-carbodiimid oder N-(3-Dimethylaminopropyl)-N'-ethylcarbodiimid in Gegenwart einer Hilfsbase, z.B. ein tertiäres Amin, zur Verbindung VII umsetzen wie beispielsweise beschrieben in der älteren Patentanmeldung PCT/EP2007/064390, auf deren Offenbarung hier ausdrücklich Bezug genommen wird.

Beispiele für Verbindungen der Formel VII, die sich nach hier beschriebenen Verfahren herstellen lassen sind:
N-(2-Bicyclopropyl-2-yl-phenyl)-3-difluormethyl-1-methylpyrazol-4-yl-carboxamid,
N-(3',4',5'-Trifluorbiphenyl-2-yl)-1,3-dimethylpyrazol-4-yl-carboxamid,
N-(3',4',5'-Trifluorbiphenyl-2-yl)-3-fluormethyl-1-methylpyrazol-4-yl-carboxamid,
N-(3',4',5'-Trifluorbiphenyl-2-yl)-3-(chlorfluormethyl)-1-methylpyrazol-4-ylcarboxamid,
N-(3',4',5'-Trifluorbiphenyl-2-yl)-3-difluormethyl-1-methylpyrazol-4-yl-carboxamid,
N-(3',4',5'-Trifluorbiphenyl-2-yl)-3-(chlordifluormethyl)-1-methylpyrazol-4-yl-carboxamid,
N-(3',4',5'-Trifluorbiphenyl-2-yl)-1-methyl-3-trifluormethyl-pyrazol-4-yl-carboxamid,
N-(2',4',5'-Trifluorbiphenyl-2-yl)-1,3-dimethylpyrazol-4-yl-carboxamid,
N-(2',4',5'-Trifluorbiphenyl-2-yl)-3-fluormethyl-1-methylpyrazol-4-yl-carboxamid,
N-(2',4',5'-Trifluorbiphenyl-2-yl)-3-(chlorfluormethyl)-1-methylpyrazol-4-yl-carboxamid,
N-(2',4',5'-Trifluorbiphenyl-2-yl)-3-difluormethyl-1-methylpyrazol-4-yl-carboxamid,
N-(2',4',5'-Trifluorbiphenyl-2-yl)-3-(chlordifluormethyl)-1-methylpyrazol-4-yl-carboxamid,
N-(2',4',5'-Trifluorbiphenyl-2-yl)-1-methyl-3-trifluormethyl-pyrazol-4-yl-carboxamid,
N-(3',4'-Dichlor-3-fluorbiphenyl-2-yl)-1-methyl-3-trifluormethyl-1H-pyrazol-4-yl-carboxamid,
N-(3',4'-Dichlor-3-fluorbiphenyl-2-yl)-1-methyl-3-difluormethyl-1H-pyrazol-4-yl-carboxamid,
N-(3',4'-Difluor-3-fluorbiphenyl-2-yl)-1-methyl-3-trifluormethyl-1H-pyrazol-4-carboxamid,
N-(3',4'-Difluor-3-fluorbiphenyl-2-yl)-1-methyl-3-difluormethyl-1H-pyrazol-4-yl-carboxamid,
N-(3'-Chlor-4'-fluor-3-fluorbiphenyl-2-yl)-1-methyl-3-difluormethyl-1H-pyrazol-4-yl-carboxamid,
N-(3',4'-Dichlor-4-fluorbiphenyl-2-yl)-1-methyl-3-trifluormethyl-1H-pyrazol-4-yl-carboxamid,
N-(3',4'-Difluor-4-fluorbiphenyl-2-yl)-1-methyl-3-trifluormethyl-1H-pyrazol-4-yl-carboxamid,
N-(3',4'-Dichlor-4-fluorbiphenyl-2-yl)-1-methyl-3-difluormethyl-1H-pyrazol-4-yl-carboxamid,
N-(3',4'-Difluor-4-fluorbiphenyl-2-yl)-1-methyl-3-difluormethyl-1H-pyrazol-4-yl-carboxamid,
N-(3'-Chlor-4'-fluor-4-fluorbiphenyl-2-yl)-1-methyl-3-difluormethyl-1H-pyrazol-4-yl-carboxamid,
N-(3',4'-Dichlor-5-fluorbiphenyl-2-yl)-1-methyl-3-trifluormethyl-1H-pyrazol-4-yl-carboxamid,
N-(3',4'-Difluor-5-fluorbiphenyl-2-yl)-1-methyl-3-trifluormethyl-1H-pyrazol-4-yl-carboxamid,
N-(3',4'-Dichlor-5-fluorbiphenyl-2-yl)-1-methyl-3-difluormethyl-1H-pyrazol-4-yl-carboxamid,
N-(3',4'-Difluor-5-fluorbiphenyl-2-yl)-1-methyl-3-difluormethyl-1H-pyrazol-4-yl-carboxamid,
N-(3',4'-Dichlor-5-fluorbiphenyl-2-yl)-1,3-dimethyl-1H-pyrazol-4-yl-carboxamid,
N-(3'-Chlor-4'-fluor-5-fluorbiphenyl-2-yl)-1-methyl-3-difluormethyl-1H-pyrazole-4-yl-carboxamid,
N-(4'-Fluor-4-fluorbiphenyl-2-yl)-1-methyl-3-trifluormethyl-1H-pyrazol-4-yl-carboxamid,
N-(4'-Fluor-5-fluorbiphenyl-2-yl)-1-methyl-3-trifluormethyl-1H-pyrazole-4-yl-carboxamid,
N-(4'-Chlor-5-fluorbiphenyl-2-yl)-1-methyl-3-trifluormethyl-1H-pyrazol-4-yl-carboxamid,
N-(4'-Methyl-5-fluorbiphenyl-2-yl)-1-methyl-3-trifluormethyl-1H-pyrazol-4-yl-carboxamid,
N-(4'-Fluor-5-fluorbiphenyl-2-yl)-1,3-dimethyl-1H-pyrazol-4-yl-carboxamid,
N-(4'-Chlor-5-fluorbiphenyl-2-yl)-1,3-dimethyl-1H-pyrazol-4-yl-carboxamid,
N-(4'-Methyl-5-fluorbiphenyl-2-yl)-1,3-dimethyl-1H-pyrazol-4-yl-carboxamid,
N-(4'-Fluor-6-fluorbiphenyl-2-yl)-1-methyl-3-trifluormethyl-1H-pyrazol-4-yl-carboxamid,
N-[2-(1,1,2,3,3,3-Hexafluorpropoxy)-phenyl]-3-difluormethyl-1-methyl-1H-pyrazol-4-yl-carboxamid,
N-[4"-(Trifluormethylthio)-biphenyl-2-yl]-3-difluormethyl-1-methyl-1H-pyrazol-4-yl-carboxamid,
N-[4'-(Trifluormethylthio)-biphenyl-2-yl]-1-methyl-3-trifluormethyl-1-methyl-1H-pyrazol-4-yl-carboxamid,
3-((Difluormethyl)-1-methyl-N-[1,2,3,4-tetrahydro-9-(1-methylethyl)-1,4-methanonaphthalin-5-yl]-1H-pyrazol-4-yl-carboxamid,
N-(3'-Chlor-5-fluorbiphenyl-2-yl)- 3-(difluormethyl)-1-methylpyrazol-4-yl-carboxamid,
N-(4'-Chlor-5-fluorbiphenyl-2-yl)- 3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid,
N-(3',5'-difluorbiphenyl-2-yl)-3-(difluormethyl)-1-methylpyrazol-4-yl-carboxamid,
N-(2-Chlor-4-fluorphenyl)- 3-(difluormethyl)-1-methylpyrazol-4-yl-carboxamid,
N-(2-Brom-4-fluorphenyl)- 3-(difluormethyl)-1-methylpyrazol-4-yl-carboxamid,
N-(2-Jod-4-fluorphenyl)-3-(difluormethyl)-1-methylpyrazol-4-yl-carboxamid und
N-[2-(1,3-Dimethylbutyl)-phenyl]-1,3-dimethyl-5-fluor-1H-pyrazol-4-yl-carboxamid.

Die folgenden Beispiele dienen der weiteren Erläuterung der Erfindung.

### Herstellungsbeispiel 1: Benzaldehydmethylhydrazon

18,4 g (0,4 mol) Methylhydrazin wurden in 248,7 g Diethylether vorgelegt. Bei 22 - 26°C wurden innerhalb von 1,75 Stunden 42,4 g (0,4 mol) Benzaldehyd zugetropft. Die Reaktionsmischung wurde dann 5 Stunden bei Rückflusstemperatur gerührt. Der nach dem Abdestillieren des Lösungsmittels erhaltene Rückstand wurde in Diethylether aufgenommen und die Lösung wurde über Natriumsulfat getrocknet. Nach der Trocknung engte man die Lösung unter vermindertem Druck ein und destillierte den erhaltenen Rückstand bei 78°C/0,5-1 mbar.

¹H-NMR (500 MHz, CDCl₃): δ (ppm) = 2,85 (s,3H), 5,55 (br., 1H), 7,2 (1 H), 7,3 (2H), 7,45 (1 H), 7,55 (2H)

In Analogie zur Vorschrift des Herstellungsbeispiels 1 wurden die folgenden Hydrazone hergestellt:

| Herstellungsbsp. | Hydrazon |
|---|---|
| 2 | o-Chlorbenzylidenmethylhydrazon |
| 3 | p-Methoxybenzylidenmethylhydrazon |
| 4 | p-Methylbenzylidenmethylhydrazon |
| 5 | o-Nitrobenzylidenmethylhydrazon |
| 6 | p-Nitrobenzylidenmethylhydrazon |
| 7 | Cyclohexylidenmethylhydrazon |

### Beispiel 1: Herstellung von 3-Difluormethyl-1-methyl-1H-pyrazol-4-carbonsäure-ethylester und anschließende Verseifung zur 3-Difluormethyl-1-methyl-1H-pyrazol-4-carbonsäure

### 1.1. 4,4-Difluor-2-[1-{N-methyl-N'-[1-phenyl-methyliden]-hydrazino}-methyliden]-3-oxo-buttersäure-ethylester

13,8 g (0,1 mol) Benzaldehyd-methylhydrazon und 62,2 g Toluol wurden mit 23,7 g (0,1 mol) 2-Ethoxymethylen-4,4-difluor-3-oxo-buttersäureethylester versetzt, wodurch die Innentemperatur auf 35°C anstieg. Die Reaktionsmischung wurde 1,25 Stunden bei Rückflusstemperatur und anschließend 15 Stunden bei 25 °C gerührt. Der ausgefallene Feststoff wurde mit einer Nutsche abgesaugt und 2-mal mit je 25 ml Toluol gewaschen. Nach dem Trocknen bei 40 - 50 °C und vermindertem Druck fielen 23 g Produkt an.
Reinheit laut HPLC: 99,2 Fl%
MS: Monoisotopische relative Molekülmasse m/z = 310
¹H-NMR (500 MHz, DMSO-d6): E/Z-Isomerengemisch (ca. 2:1) bezüglich der C=C-Doppelbindung: δ (ppm) = 1,07 und 2,2 (3H), 3,55 und 3,62 (3H), 4, 08 - 4,2 (2H), 6,15 und 6,7 (t, 1H,-CHF₂-), 7,4 - 7,75 (5H), 7,93 (1H), 8,05 und 8,13 (1 H)
¹³C-NMR: 190,1, 181,4, 166,6, 164,7, 148,9, 146,1, 145,5, 133,9, 130,3, 128,8, 127,7, 110,4, 108,5, 107,0, 99,23, 60,46, 59,66, 39,43, 13,81.

### 1.2. 3-Difluormethyl-1-methyl-1 H-pyrazol-4-carbonsäure

20 g (0,065 mol) 4,4-Difluor-2-[1-{N-methyl-N'-[1-phenyl-methyliden]-hydrazino}-methyliden]-3-oxo-buttersäure-ethylester aus Schritt 1.1. wurden zusammen mit 252,7 g Ethanol unter Stickstoffatmosphäre bei 25 °C vorgelegt. Innerhalb von 5 Minuten wurden 14,8 g (0,13 mol) Salzsäure (32 %ig) zugetropft. Die Suspension wurde auf 45 °C erwärmt und 30 Minuten bei Umgebungstemperatur weitergerührt. Danach lag eine gelbe klare Lösung vor. Die Lösung (285 g) enthielt 4,12 Gew.-% des gewünschten 3-Difluormethyl-1-methyl-1H-pyrazol-4-carbonsäure-ethylesters (HPLC-Analytik, Quantifizierung mit externem Standard), entsprechend einer Ausbeute von 89,2 %. Der Anteil an dem isomeren 5-Difluormethyl-1-methyl-1 H-pyrazol-4-carbonsäure-ethylester betrug lediglich 0,05 Gew.-% (I-somerenverhältnis ca. 82:1).

Bei 25 - 27 °C wurden nun 104g (0,26 mol) 10 %ige Natronlauge innerhalb von 5 Minuten zudosiert und es wurde mit 50 ml Wasser nachgespült. Die Reaktionsmischung wurde 2,5 Stunden bei 60 °C gerührt. Bei 58 °C/370 mbar destilliert man 320 g Lösungsmittel (Ethanol/Wasser) ab, wobei ein zweiphasiger Destillationssumpf zurückblieb. Nach Verdünnung mit 100 ml Toluol wurden die Phasen getrennt. Die toluolische Oberphase enthielt hauptsächlich den freigesetzten Benzaldehyd. Die untere wässrige Phase enthielt als Hauptkomponente das Natriumsalz der gewünschten 3-Difluormethyl-1-methyl-1 H-pyrazol-4-carbonsäure. Die abgetrennte wässrige Phase wurde mit 29,7 g (0,26 mol) konzentrierter Salzsäure angesäuert (pH < 2), wobei die Titelverbindung ausfiel. Nach Filtration erhielt man 18,2 g der feuchten 3-Difluormethyl-1-methyl-1 H-pyrazol-4-carbonsäure. Die HPLC-Analytik (Quantifizierung mit externem Standard) ergab einen Gehalt von 52,6 Gew.-%, entsprechend einer Ausbeute von 83%, bezogen auf den zur Reaktion eingesetzten 4,4-Difluor-2-[1-{N-methyl-N'-[1-phenyl-methyliden]-hydrazino}-methyliden]-3-oxo-buttersäure-ethylester.

### Beispiel 2: Herstellung von 3-Difluormethyl-1-methyl-1H-pyrazol-4-carbonsäureethylester mit einer katalytischen Menge an p-Toluolsulfonsäure und anschließende Verseifung zur 3-Difluormethyl-1-methyl-1 H-pyrazol-4-carbonsäure

62 g (0,2 mol) 4,4-Difluor-2-[1-{N-methyl-N'-[1-phenyl-methyliden]-hydrazino}-methyliden]-3-oxo-buttersäure-ethylester (analog zu Beispiel1, Schritt 1.1. hergestellt, Reinheit 99,1 FI%) wurden zusammen mit 150 g Ethanol unter Stickstoffatmosphäre bei 15 °C vorgelegt. Man gab 1,6 g (0,0083 mol) p-Toluolsulfonsäure-Monohydrat hinzu und rührte 15 Stunden bei 25 °C und 1 Stunde bei 50 °C. Die Lösung enthielt 14,9 Gew.-% des gewünschten 3-Difluormethyl-1-methyl-1H-pyrazol-4-carbonsäure-ethylesters (HPLC-Analytik, Quantifizierung mit externem Standard). Der Anteil an dem isomeren 5-Difluormethyl-1-methyl-1 H-pyrazol-4-carbonsäure-ethylester beträgt nur 0,069 Gew.-% (entsprechend einem Isomerenverhältnis von > 200: 1).

Es wurden nun 168,3 g (0,3 mol) 10 %ige Kalilauge zudosiert und die Reaktionsmischung wurde 3 Stunden bei 60 °C gerührt. Nach Abkühlen auf 25 °C wurden die Phasen getrennt. Die toluolische Oberphase enthielt hauptsächlich den freigesetzten Benzaldehyd. Die untere wässrige Phase enthielt als Hauptkomponente das Kaliumsalz der gewünschten 3-Difluormethyl-1-methyl-1 H-pyrazol-4-carbonsäure. Die Toluolphase wurde noch zweimal mit jeweils 50 g Wasser gewaschen. Die vereinigten Wasserphasen wurden bei 55 °C mit 66 g (0,579 mol) konzentrierter Salzsäure (32 %ig) angesäuert (pH < 2), wobei die gewünschte Titelverbindung ausfiel. Der Feststoff wurde bei 3 °C abfiltriert und mit 132 g kaltem Wasser gewaschen. Nach der Trocknung (60 °C, 20 mbar) erhielt man 32,1 g 3-Difluormethyl-1-methyl-1 H-pyrazol-4-carbonsäure in einer Reinheit von 99 Gew.-% an. Die Ausbeute bezogen auf die eingesetzte Molmenge Methylhydrazin bzw. 2-Ethoxymethylen-4,4-difluor-3-oxo-buttersäure-ethylester betrug 90,3 %. Das unerwünschte 1,5-Isomer ist nicht mehr nachweisbar.

### Beispiel 3: Herstellung von 3-Difluormethyl-1-methyl-1 H-pyrazol-4-carbonsäure aus Benzaldehyd, Methylhydrazin und Ethoxymethylen-4,4-difluor-3-oxo-buttersäure-ethylester ohne Isolierung/Reinigung der Zwischenprodukte (Eintopffahrweise)

9,4 g (0,2 mol) Methylhydrazin (98 %ig) wurden in 150,2 g Toluol vorgelegt. Bei 22 - 26 °C wurden innerhalb von 10 Minuten 21,4 g (0,2 mol) Benzaldehyd zugetropft. Anschließend wurde auf 40 °C aufgeheizt und der Fortgang der Umsetzung mittels GC-Analytik verfolgt. Nach 8 Stunden war kein Benzaldehyd mehr nachweisbar. Die Wasserphase wurde abgetrennt. Von der Toluolphase, enthaltend das Hydrazon, wurde bei 40 °C und reduziertem Druck soviel Lösungsmittel abdestilliert, bis die Lösung klar wurde (Entfernung von restlichem Wasser).

Die verbleibende Lösung (91,1 g) wurde auf 3 °C abgekühlt. Bei dieser Temperatur tropfte man 45,7 g (0,2 mol) 2-Ethoxymethylen-4,4-difluor-3-oxo-buttersäure-ethylester (97,1 %ig) als Lösung in 60 g Toluol zu. Nach dem Aufheizen auf 25 °C rührte man 15 Stunden bei dieser Temperatur nach. Es entstand eine hellgelbe Suspension (ausgefallener 4,4-Difluor-2-[1-{N-methyl-N'-[1-phenyl-methyliden]-hydrazino}-methyliden]-3-oxo-buttersäure-ethylester).

Zu der Suspension gab man 1,7 g p-Toluolsulfonsäure-Monohydrat (0,009 mol) und rührte 1 Stunde bei 70°C, wobei eine klare Lösung entsteht. Nach HPLC-Analytik (Quantifizierung mit externem Standard) lagen 15,2 Gew.-% des gewünschten 3-Difluormethyl-1-methyl-1 H-pyrazol-4-carbonsäure-ethylester und nur 0,164 Gew.-% des unerwünschten 5-Difluormethyl-1-methyl-1 H-pyrazol-4-carbonsäure-ethylester vor (entsprechend einem Isomerenverhältnis von > 92: 1).

Zu der Lösung gab man 168,3 g 10 %ige Kalilauge (0,3 mol) und rührte die Mischung 3 Stunden bei 60 °C. Nach Abkühlen auf 25°C wurden die Phasen getrennt. Die toluolische Oberphase enthielt hauptsächlich den freigesetzten Benzaldehyd. Die untere wässrige Phase enthielt als Hauptkomponente das Kaliumsalz der gewünschten 3-Difluormethyl-1-methyl-1 H-pyrazol-4-carbonsäure. Die Toluolphase wurde noch zweimal mit jeweils 50 g Wasser gewaschen. Die vereinigten Wasserphasen wurden bei 55 °C mit 66 g (0,579 mol) konz. Salzsäure (32%ig) angesäuert (pH < 2), wobei die gewünschte Carbonsäure ausfällt. Der Feststoff wurde bei 3°C abfiltriert und mit 132 g kaltem Wasser gewaschen. Nach der Trocknung (60 °C, 20 mbar) fallen 30,6 g 3-Difluormethyl-1-methyl-1 H-pyrazol-4-carbonsäure in einer Reinheit von 98,6 Gew% an. Die Ausbeute bezogen auf die eingesetzte Molmenge Methylhydrazin bzw. 2-Ethoxymethylen-4,4-difluor-3-oxo-buttersäure-ethylester beträgt 85,7%. Das unerwünschte Carbonsäure-Isomer ist nicht mehr enthalten.

### Beispiel 4: Herstellung von 3-Difluormethyl-1-methyl-1 H-pyrazol-4-carbonsäure aus Aceton, Methylhydrazin und Ethoxymethylen-4,4-difluor-3-oxo-buttersäure-ethylester ohne Isolierung/Reinigung der Zwischenprodukte (Eintopffahrweise)

11,5 g (0,245 mol) Methylhydrazin (98%ig) wurden in 150 g Toluol vorgelegt. Bei 0 - 5 °C wurden innerhalb von 10 Minuten 15,1 g (0,258 mol) Aceton zugetropft. Man rührt 1 Stunde bei 5 °C nach. Bis zu einer Innentemperatur von 100 °C wurde nun Toluol/Wasser abdestilliert. Man erhielt auf diese Weise 163,1 g einer Lösung von Aceton-methylhydrazon in Toluol.

Zu 163,1 g Aceton-methylhydrazon-Lösung wurde bei 23 °C eine Lösung aus 56,9 g (0,24 mol) 2-Ethoxymethylen-4,4-difluor-3-oxo-buttersäure-ethylester (93,7 %ig) und 60 g Toluol innerhalb von 10 Minuten zudosiert. Man rührte 1 Stunde bei 3 °C nach. Bei 40 °C und reduziertem Druck wurden 100 g Lösungsmittel abdestilliert und wieder 100 g frisches Toluol zudosiert. Bei 15 °C gab man 2 g (0,01 mol) p-Toluolsulfonsäure-Monohydrat hinzu, wobei die Innentemperatur bis auf 35 °C anstieg. Nach Abkühlen auf 25 °C wurde noch 1 Stunde bei dieser Temperatur gerührt. Nach HPLC-Analytik (Quantifizierung mit externem Standard) liegen 11,3 Gew.-% des gewünschten 3-Difluormethyl-1-methyl-1 H-pyrazol-4-carbonsäure-ethylester und nur 0,064 Gew.-% des unerwünschten 5-Difluormethyl-1-methyl-1 H-pyrazol-4-carbonsäure-ethylester vor. (entsprechend einem Isomerenverhältnis von > 175: 1)

Zu der Lösung gab man 202 g 10 %ige Kalilauge (0,361 mol) und rührte die Mischung 3 Stunden bei 60 °C. Nach Abkühlen auf 25 °C wurden die Phasen getrennt. Die toluolische Oberphase enthielt hauptsächlich den freigesetzten Benzaldehyd. Die untere wässrige Phase enthielt als Hauptkomponente das Kaliumsalz der Titelverbindung. Die Toluolphase wurde noch zweimal mit jeweils 50 g Wasser gewaschen. Die vereinigten Wasserphasen wurden bei 55 °C mit 80 g (0,7 mol) konzentrierter Salzsäure (32 %ig) angesäuert (pH < 2), wobei die gewünschte Pyrazolcarbonsäure ausfiel. Der Feststoff wurde bei 3 °C abfiltriert und mit 160 g kaltem Wasser gewaschen. Nach der Trocknung (60 °C, 20 mbar) erhielt man 34,6 g 3-Difluormethyl-1-methyl-1H-pyrazol-4-carbonsäure in einer Reinheit von 99 Gew.-% an. Die Ausbeute bezogen auf die eingesetzte Molmenge 2-Ethoxymethylen-4,4-difluor-3-oxo-buttersäure-ethylester betrug 81 %. Das unerwünschte 1,5-Isomer war nicht mehr nachweisbar.

### Beispiel 5: Herstellung von 3-Difluormethyl-1-methylpyrazol-4-carbonsäuremethylester aus 1,1,2,2-Tetrafluorethyldimethylamin, 3-Methoxyacrylsäuremethylester und N-Methylbenzaldehydhydrazon

Zu einer Lösung von 96 %igem 1,1,2,2-Tetrafluorethyldimethylamin (48,1 g, 318 mmol) in Acetonitril (97 g) unter Argon tropfte man bei 25 °C 38,4 g (270 mmol) BF₃-Etherat. Nach beendeter Zugabe erhitzte man zum Rückfluss (70 °C). Bei dieser Temperatur tropfte man zu dem Reaktionsgemisch innerhalb von 1 h eine Lösung von 95%igem 3-Methoxyacrylsäuremethylester (33,1 g, 271 mmol) in Acetonitril (61 g). Nach 20 h Rühren unter Rückfluss kühlte man das Reaktionsgemisch auf 25 °C und gab 99,8 g einer 38 %igen Lösung von N-Methyl-benzaldehydhydrazon in Toluol (287 mmol) bei 25 °C innerhalb von 15 min zu. Nach einer Nachrührphase von 0,5 h gab man 10,4 g einer 50 gew.-%igen Lösung von Wasser in Acetonitril (289 mmol). Dann gab man 32,7 g (287 mmol) 32 %ige Salzsäure zu und erhitzte die Mischung unter Rühren 3h zum Rückfluss. Anschließend wurde auf 25° C abgekühlt und 100 ml Wasser zugegeben. Die organische Phase wurde abgetrennt, die Wasserphase wurde einmal mit 100 ml Methylenchlorid extrahiert. Die vereinigten organischen Phasen wurden einmal mit 100 ml Wasser gewaschen. Es wurden 391 g organische Phase erhalten. Gaschromatographische Analyse zeigte, dass das unerwünschte 1,5-Isomer (5-Difluormethyl-1-methylpyrazol-4-carbonsäuremethylester) neben dem 3-Difluormethyl-1-methylpyrazol-4-carbonsäuremethylester nur in Spuren gebildet wurde. Das Isomerenverhältnis lag bei 141: 1. Die organische Phase wurde aufkonzentriert. Man erhielt 63,6 g Rückstand, der neben Benzaldehyd It. quantitativer HPLC-Analytik zu 71,7 Gew.-% 3-Difluormethyl-1-methylpyrazol-4-carbonsäuremethylester enthielt. Dies entspricht 89% Ausbeute bezogen auf 3-Methoxyacrylsäuremethylester. Der Benzaldehyd kann leicht durch fraktionierte Destillation oder nach Verseifung der Titelverbindung wie in den Beispielen 1 bis 4 beschrieben, abgetrennt werden.

### Beispiel 6: Herstellung von 3-Difluormethyl-1-methyl-1 H-pyrazol-4-carbonsäure aus Benzaldehyd, wässriger Methylhydrazin-Lösung und Ethoxymethylen-4,4-difluor-3-oxo-buttersäure-ethylester ohne Isolierung/Reinigung der Zwischenprodukte (Eintopffahrweise)

108,2 g (0,816 mol) Methylhydrazin-Lösung (34,7 Gew.-% Methylhydrazin in Wasser) und 560 g Toluol wurden im Rührbehälter unter Stickstoffatmosphäre vorgelegt. Bei 25-40°C tropfte man innerhalb von 10 Minuten 85,7 g (0,8 mol) Benzaldehyd (99%ig) hinzu. Die Reaktionsmischung wurde 3 Stunden bei 40°C und 3 Stunden bei 60°C gerührt. Anschließend wurde bei 70°C/150mbar Toluol/Wasser abdestilliert, wobei das Wasser des kondensierten Destillats in einem Phasenscheider abgetrennt und die Toluolphase in den Reaktor zurückgeführt wurde. Nach der Wasserauskreisung verblieben 656 g einer klaren Lösung von Benzaldehyd-methylhydrazon in Toluol.

Zu dieser Lösung tropfte man innerhalb von 1 Stunde bei 20 - 30 °C 189,5 g (0,8 mol) 2-Ethoxymethylen-4,4-difluor-3-oxo-buttersäure-ethylester (93,7%ig) als Lösung in 189,5 g Toluol. Man rührte 18 Stunden bei 25 °C nach. Es bildete sich eine Suspension (ausgefallener 4,4-Difluor-2-[1-{N-methyl-N'-[1-phenyl-methyliden]-hydrazino}-methyliden]-3-oxo-buttersäure-ethylester).

Zu der Suspension gab man bei 10 °C 6,2 g p-Toluolsulfonsäure-Monohydrat (0,032 mol) und rührte 1 Stunde bei 50 °C, wobei eine klare Lösung entstand. Nach HPLC-Analytik (Quantifizierung mit externem Standard) betrug die Konzentration des gewünschten 3-Difluormethyl-1-methyl-1 H-pyrazol-4-carbonsäure-ethylester 11,4 Gew.-%.

Zu der Lösung gab man 672 g 10 %ige Kalilauge (1,2 mol) und rührte die Mischung 3 Stunden bei 60 °C. Nach Abkühlen auf 25 °C wurden die Phasen getrennt. Die toluolische Oberphase enthielt hauptsächlich den freigesetzten Benzaldehyd. Die untere wässrige Phase enthielt als Hauptkomponente das Kaliumsalz der gewünschten 3-Difluormethyl-1-methyl-1 H-pyrazol-4-carbonsäure. Die Toluolphase wurde noch zweimal mit jeweils 200 g Wasser gewaschen. Die vereinigten Wasserphasen wurden bei 55 °C mit 265 g (2,32 mol) konz. Salzsäure (32 %ig) angesäuert (pH < 2), wobei die gewünschte Pyrazolcarbonsäure ausfiel. Der Feststoff wurde bei 3 °C abfiltriert und zweimal mit jeweils 265 g kaltem Wasser gewaschen. Nach der Trocknung (60 °C, 20 mbar) erhielt man 121,8 g 3-Difluormethyl-1-methyl-1 H-pyrazol-4-carbonsäure in einer Reinheit von 99,5 Gew.-% an. Die Ausbeute, bezogen auf die eingesetzte Molmenge Benzaldehyd bzw. 2-Ethoxymethylen-4,4-difluor-3-oxo-buttersäure-ethylester, betrug 86,1 %. Das unerwünschte Carbonsäure-Isomer war nicht mehr nachweisbar.

### Beispiel 7: Herstellung von 3-Difluormethyl-1-methyl-1 H-pyrazol-4-carbonsäure aus Benzaldehyd, wässriger Methylhydrazin-Lösung und Ethoxymethylen-4,4-difluor-3-oxo-buttersäure-ethylester ohne Isolierung/Reinigung der Zwischenprodukte (Eintopffahrweise).

368,3 g (2,78 mol) Methylhydrazin-Lösung (34,7 Gew.-% Methylhydrazin in Wasser) und 1888 g Toluol wurden im Rührbehälter unter Stickstoffatmosphäre vorgelegt. Die Reaktionsmischung wurde auf 40°C erhitzt. Bei 40°C bis 60°C gab man innerhalb von 30 Minuten 300,9 g (2,81 mol) Benzaldehyd (99%ig) hinzu. Die Reaktionsmischung wurde 4 Stunden bei 60°C gerührt. Nach Abkühlen auf 25°C wurde die untere wässrige Phase abgetrennt. Von der im Reaktor verbliebenen organische Phase wurden bei 25 bis 45°C und einem Druck von 100 mbar ca. 99 g Toluol/Wasser abdestilliert (azeotrope Trocknung). Nach der Destillation ergänzte man wieder 99 g frisches Toluol. Es verblieben ca. 2282 g einer klaren Lösung von Benzaldehydmethylhydrazon in Toluol.

Zu dieser Lösung gab man innerhalb von 2 Stunden bei 25 bis 30°C 635,6 g (2,70 mol) 2-Ethoxymethylen-4,4-difluor-3-oxo-buttersäureethylester (94,2 gew.-%ig) und rührte 1 Stunde bei 30°C nach. Die erhaltene Lösung enthielt 27,8 Gew.-% des gewünschten 4,4-Difluor-2-[1-{N-methyl-N'-[1-phenyl-methyliden]-hydrazino}-methyliden]-3-oxo-buttersäure-ethylesters (HPLC-Analytik).

Zu der Lösung dosierte man bei 60°C 1620 g (4,05 mol) 10 gew.-%ige Natronlauge und rührte die Mischung 3 Stunden bei 60°C. Nach Abkühlen auf 25°C wurden die Phasen getrennt. Die toluolische Oberphase enthielt hauptsächlich den freigesetzten Benzaldehyd. Die untere wässrige Phase enthielt als Hauptkomponente das Natriumsalz der gewünschten 3-Difluormethyl-1-methyl-1 H-pyrazol-4-carbonsäure. Die Toluolphase wurde noch mit 540 g Wasser gewaschen. Zu den vereinigten Wasserphasen gab man weitere 1125 g Wasser. Zu der wässrigen Carboxylat-Lösung wurden dann bei 53 bis 56°C innerhalb von 30 Minuten 1277,5 g (3,91 mol) Schwefelsäure (30%ig in Wasser) gegeben, wobei die gewünschte Pyrazolcarbonsäure ausfiel. Nach dem Abkühlen auf 3°C wurde der Feststoff abfiltriert und portionsweise mit insgesamt 1880 g Wasser (25°C) gewaschen. Nach der Trocknung (60°C, 20 mbar) erhielt man 402,2 g 3-Difluormethyl-1-methyl-1H-pyrazol-4-carbonsäure in einer Reinheit von 99,4 Gew% an. Die Ausbeute bezogen auf die eingesetzte Molmenge 2-Ethoxymethylen-4,4-difluor-3-oxo-buttersäure-ethylester betrug 84,2%. Das unerwünschte Carbonsäure-Isomer war nicht mehr nachweisbar.

### Beispiel 8: Herstellung von 3-Difluormethyl-1-methyl-1H-pyrazol-4-carbonsäure aus p-Chlorbenzaldehyd, Methylhydrazin und Ethoxymethylen-4,4-difluor-3-oxo-buttersäureethylester ohne Isolierung/Reinigung der Zwischenprodukte (Eintopffahrweise)

9,4 g (0,2 mol) Methylhydrazin (98%ig) wurden in 150,2 g Toluol vorgelegt. Bei Raumtemperatur wurden innerhalb von 10 Minuten 28,11 g (0,2 mol) p-Chlorbenzaldehyd zugegeben, so dass die Temperatur auf 45 bis 50 °C anstieg. Anschließend wurde 1 Stunde bei 60°C nachgerührt. Die Wasserphase wurde abgetrennt. Von der Toluolphase, enthaltend das Hydrazon, wurde bei 40°C und reduziertem Druck soviel Lösungsmittel abdestilliert, bis die Lösung klar wurde (Entfernung von restlichem Wasser).

Die verbleibende Lösung wurde auf die ursprüngliche Gesamtmasse mit Toluol aufgefüllt und auf 3°C abgekühlt. Bei 3 bis 6 °C gab man hierzu innerhalb von 45 min. 47,4 g (0,2 mol) 2-Ethoxymethylen-4,4-difluor-3-oxo-buttersäure-ethylester (93,8 %ig). Man erwärmte auf 25°C und rührte 15 Stunden bei dieser Temperatur nach. Es entstand eine hellgelbe Suspension (ausgefallener 4,4-Difluor-2-[1-{N-methyl-N'-[1-(4-Chlorphenyl)-methyliden]-hydrazino}-methyliden]-3-oxo-buttersäure-ethylester.

Zu der Suspension gab man 1,8 g p-Toluolsulfonsäure-Monohydrat (0,009 mol) und rührte 1 Stunde bei 70°C, wobei eine klare Lösung entsteht. Zu der Lösung gab man 250 g 10%ige Kalilauge (0,45 mol) und rührte die Mischung 3 Stunden bei 60°C. Nach Abkühlen auf 25°C wurden die Phasen getrennt. Die Toluolphase wurde noch zweimal mit jeweils 50 g Wasser gewaschen. Die vereinigten Wasserphasen wurden bei 50°C mit 60 g (0,52 mol) konzentrierter Salzsäure (32 gew.-%ig) angesäuert (pH < 2), wobei die gewünschte Carbonsäure ausfiel. Der Feststoff wurde bei 10°C abfiltriert und mit kaltem Wasser gewaschen. Nach der Trocknung (60°C, 20 mbar) fielen 26,3 g 3-Difluormethyl-1-methyl-1 H-pyrazol-4-carbonsäure in einer Reinheit von 93,3 Gew% an. Die Ausbeute bezogen auf die eingesetzte Molmenge Methylhydrazin bzw. 2-Ethoxymethylen-4,4-difluor-3-oxo-buttersäure-ethylester beträgt 75,5 %.

Analog zu Beispiel 8 wurde die Synthese von 3-Difluormethyl-1-methyl-1 H-pyrazol-4-carbonsäure mittels entsprechend substituierter Benzaldehyde und Ketone durchgeführt:

| **Beispiel** | **Benzaldehyd/Keton** | **Ausbeute [%[** | **Reinheit [Gew%]** |
|---|---|---|---|
| 9 | o-Chlorbenzaldehyd | 67,2 | 98,5 |
| 10 | p-Methoxybenzaldehyd | 52,1 | 96,5 |
| 11 | p-Methylbenzaldehyd | 70,7 | 100 |
| 12 | o-Nitrobenzaldehyd¹ | 52,1 | n.b. |
| 13 | p-Nitrobenzaldehyd¹ | 58,1 | n.b. |
| 14 | Cyclohexanon | 71,5 | 100 |

| | | | |
|---|---|---|---|
| ¹ Die Umsetzung des Nitrobenzaldehys mit Methylhydrazin zum entsprechenden Hydrazon wurde nicht bis zum vollständigen Umsatz geführt. Daher ist das Endprodukt durch Nitrobenzaldehyd verunreinigt, der aus wässrigen Lösungen zusammen mit 3-Difluormethyl-1-methyl-1 H-pyrazol-4-carbonsäure als Feststoff ausfiel. | | | |

In Analogie zur Herstellung von 4,4-Difluor-2-[1-{N-methyl-N'-[1-phenyl-methyliden]-hydrazino}-methyliden]-3-oxo-buttersäureethylester (Beispiel 1, Schritt 1.1) wurden die folgenden Verbindungen der Formel VI hergestellt:

### Beispiel 15: 4,4-Difluor-2-[1-{N-methyl-N'-[1-(4-chlorphenyl)methyliden]-hydrazino}-methyliden]-3-oxo-buttersäureethylester

¹³C-NMR: 190,2, 181,5, 166,6, 164,6, 148,8, 144,8, 144,2, 135,2, 132,8, 128,9, 128,6, 110,4, 108,4, 107,2, 99,60, 60,51, 59,71, 40,08, 13,86.

### Beispiel 16: 4,4-Difluor-2-[1-{N-methyl-N'-[1-(2-chlorphenyl)methyliden]-hydrazino}-methyliden]-3-oxo-buttersäureethylester

¹³C-NMR: 190,3, 181,6, 166,4, 164,5, 148,6, 140,9, 140,2, 133,5, 132,1, 131,0, 130,0, 127,5, 127,4, 110,2, 108,3, 107,7, 100,3, 60,46, 59,77, 39,94, 13,74.

### Beispiel 17: 4,4-Difluor-2-[1-{N-methyl-N'-[1-(4-methoxyphenyl)methyliden]-hydrazino}-methyliden]-3-oxo-buttersäureethylester

¹³C-NMR: 190,8, 181,1, 166,8, 164,8, 161,3, 148,7, 146,1, 145,5, 129,5, 126,4, 114,3, 110,4, 108,5, 106,4, 98,46, 60,36, 59,54, 55,30, 39,36, 13,86.

### Beispiel 18: 4,4-Difluor-2-[1-{N-methyl-N'-[1-(4-methylphenyl)methyliden]-hydrazino}-methyliden]-3-oxo-buttersäureethylester

¹³C-NMR: 189,9, 181,2, 166,6, 164,6, 148,8, 146,1, 145,5, 140,7, 131,1, 129,4, 109,3, 108,4, 106,7, 98,82, 60,34, 59,54, 39,43, 21,00, 13,81.

### Beispiel 19: 4,4-Difluor-2-[1-{N-methyl-N'-[1-(2-nitrophenyl)methyliden]-hydrazino}-methyliden]-3-oxo-buttersäureethylester

¹³C-NMR: 190,8, 181,8, 164,5, 164,4, 148,6, 148,3, 141,5, 140,6, 133,7, 132,8, 131,2, 128,6, 124,9, 110,2, 108,3, 108,1, 100,8, 60,51, 59,66, 39,33, 13,69.

### Beispiel 20: 4,4-Difluor-2-[1-{N-methyl-N'-[1-(4-nitrophenyl)methyliden]-hydrazino}-methyliden]-3-oxo-buttersäureethylester

¹³C-NMR: 190,3, 181,8, 164,4, 148,1, 143,5, 142,9, 140,0, 139,8, 128,4, 124,0, 110,3, 108,2, 108,1, 100,8, 60,67, 59,89, 39,65, 14,15.

Die Verbindungen der Beispiele 15 bis 20 wurden in Analogie zu Beispiel 1, Schritt 1.2 zum 3-Difluormethyl-1-methyl-1 H-pyrazol-4-carbonsäureethylester umgesetzt, welcher anschließend zur 3-Difluormethyl-1-methyl-1 H-pyrazol-4-carbonsäure verseift wurde.

## Patentansprüche

1. Verfahren zur Herstellung von 1,3,4-substituierten Pyrazolverbindungen der Formel I, 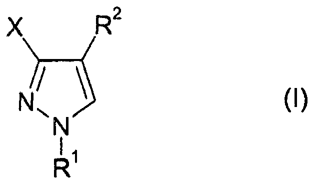 worin
X für eine Gruppe CX¹X²X³ steht, worin
X¹, X² und X³ unabhängig voneinander für Wasserstoff, Fluor oder Chlor stehen, wobei X¹ auch für C₁-C₆-Alkyl oder C₁-C₄-Haloalkyl stehen kann und wobei wenigstens einer der Reste X¹, X² von Wasserstoff verschieden ist,
R¹ für Methyl steht, und
R² für eine Gruppe CO₂R^{2a} steht, worin
R^{2a} für C₅-C₆-Cycloalkyl, gegebenenfalls substituiertes Phenyl oder C₁-C₆-Alkyl steht, das gegebenenfalls durch C₁-C₄-Alkoxy, Phenyl oder C₃-C₆-Cycloalkyl substituiert sein kann,
umfassend die folgenden Schritte:
i) Umsetzung einer Verbindung der Formel II mit einem Hydrazon der Formel III, 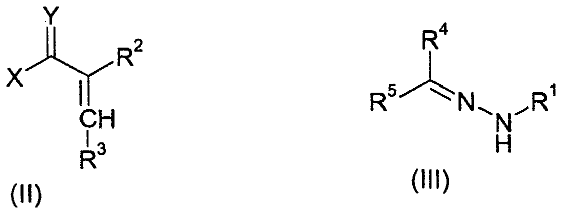 wobei in Formel II die Variablen X und R² die für Formel I angegebenen Bedeutungen aufweisen,
Y für Sauerstoff, eine Gruppe NR^{y1} oder eine Gruppe [NR^{y2}R^{y3}]+Z- steht, worin
R^{y1}, R^{y2} und R^{y3} unabhängig voneinander für C₁-C₆-Alkyl, C₅-C₆-Cycloalkyl, gegebenenfalls substituiertes Phenyl oder gegebenenfalls substituiertes Phenyl-C₁-C₄-alkyl stehen, oder
R^{y2} und R^{y3} zusammen mit dem Stickstoffatom, an das diese gebunden sind, für einen N-gebundenen 5- bis 8-gliedrigen, gesättigten, gegebenenfalls substituierten Heterocyclus stehen, der neben dem Stickstoffatom noch 1 oder 2 weitere Heteroatome ausgewählt unter N, O und S als Ringatome aufweisen kann, und Z- für einen Anion steht;
R³ für OR^{3a} oder eine Gruppe NR^{3b}R^{3c} steht, worin
R^{3a}, R^{3b} und R^{3c} unabhängig voneinander für C₁-C₆-Alkyl, C₅-C₆-Cycloalkyl, gegebenenfalls substituiertes Phenyl oder gegebenenfalls substituiertes Phenyl-C₁-C₄-alkyl stehen, stehen, oder
R^{3b} und R^{3c} zusammen mit dem Stickstoffatom, an das diese gebunden sind, für einen N-gebundenen 5- bis 8-gliedrigen, gesättigten, gegebenenfalls substituierten Heterocyclus stehen, der neben dem Stickstoffatom noch 1 oder 2 weitere Heteroatome ausgewählt unter N, O und S als Ringatome aufweisen kann,
und wobei in Formel III die Variable R¹ die für Formel I angegebenen Bedeutungen aufweist,
R⁴ und R⁵ unabhängig voneinander für Wasserstoff, C₁-C₆-Alkyl, das gegebenenfalls durch C₁-C₄-Alkoxy, Phenyl oder C₃-C₆-Cycloalkyl substituiert sein kann, C₃-C₆-Cycloalkyl oder gegebenenfalls substituiertes Phenyl stehen, wobei wenigstens einer der Reste R⁴ oder R⁵ von Wasserstoff verschieden ist, und wobei
R⁴ und R⁵ gemeinsam mit dem Kohlenstoffatom, an das sie gebunden sind, auch für einen 5- bis 10-gliedrigen gesättigten Carbocyclus stehen können, der gegebenenfalls ein- oder mehrfach durch C₁-C₄-Alkylgruppen und/oder gegebenenfalls substituiertes Phenyl substituiert ist und/oder einen oder 2 kondensierte Phenylringe aufweist;
ii) Behandlung des dabei erhaltenen Reaktionsprodukts mit einer Säure in Gegenwart von Wasser.

2. Verfahren nach Anspruch 1, umfassend zusätzlich die Herstellung der Verbindung III durch Umsetzung einer Carbonylverbindung der Formel IV mit einer substituierten Hydrazinverbindung der Formel V 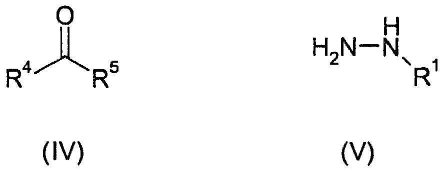 wobei R¹, R⁴ und R⁵ in den Formeln IV und V die für Formel III angegebenen Bedeutungen aufweisen.

3. Verfahren nach einem der vorhergehenden Ansprüche, worin R³ in Formel II für O-R^{3a} steht, worin R^{3a} die zuvor genannten Bedeutungen aufweist und insbesondere für C₁-C₄-Alkyl steht.

4. Verfahren nach einem der vorhergehenden Ansprüche, worin Y in Formel II für Sauerstoff steht.

5. Verfahren nach einem der vorhergehenden Ansprüche, wobei X in den Formeln I und II für eine Gruppe CX¹X²X³ steht, worin X¹ und X² für Fluor stehen und X³ für Wasserstoff, Fluor oder Chlor steht.

6. Verfahren nach einem der vorhergehenden Ansprüche, wobei R^{2a} in der Gruppe COOR^{2a} für C₁-C₆-Alkyl oder C₁-C₄-Alkoxy-C₁-C₆-alkyl steht.

7. Verfahren nach einem der vorhergehenden Ansprüche, worin
R⁴ für Wasserstoff oder C₁-C₆-Alkyl steht und
R⁵ für C₁-C₆-Alkyl, C₃-C₆-Cycloalkyl oder gegebenenfalls substituiertes Phenyl steht, oder
R⁴ und R⁵ gemeinsam mit dem Kohlenstoffatom, an das sie gebunden sind, für einen 5- bis 10-gliedrigen gesättigten Carbocyclus stehen können, der gegebenenfalls ein- oder mehrfach durch C₁-C₄-Alkylgruppen substituiert ist und/oder einen kondensierten Phenylring aufweist.

8. Verfahren nach einem der vorhergehenden Ansprüche, worin
R⁴ für Wasserstoff steht und
R⁵ für gegebenenfalls substituiertes Phenyl steht.

9. Verbindung der allgemeinen Formel VI 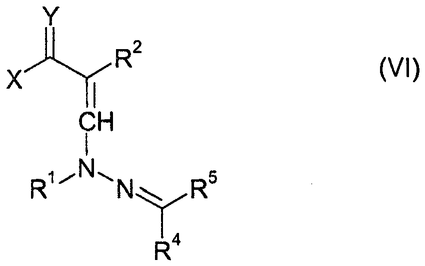 worin X, Y, R¹, R², R⁴ und R⁵ die in Anspruch 1 angegebenen Bedeutungen aufweisen, ausgenommen Verbindungen der Formel VI, worin R⁴ und R⁵ jeweils für gegebenenfalls substituiertes Phenyl stehen und Y für Sauerstoff steht.

10. Verbindung nach Anspruch 9, worin Y für Sauerstoff steht;
X für eine Gruppe CX¹X²X³ steht, worin X¹ und X² für Fluor stehen und X³ für Wasserstoff, Fluor oder Chlor steht;
R¹ für C₁-C₄-Alkyl und insbesondere für Methyl steht;
R² für eine Gruppe COOR^{2a} steht, worin R^{2a} die in Anspruch 1 genannten Bedeutungen aufweist und insbesondere für C₁-C₆-Alkyl oder C₁-C₄-Alkoxy-C₁-C₆-alkyl steht,
R⁴ für Wasserstoff oder C₁-C₆-Alkyl steht und
R⁵ für C₁-C₆-Alkyl, C₃-C₆-Cycloalkyl oder gegebenenfalls substituiertes Phenyl steht, oder
R⁴ und R⁵ gemeinsam mit dem Kohlenstoffatom, an das sie gebunden sind, für einen 5- bis 10-gliedrigen gesättigten Carbocyclus stehen können, der gegebenenfalls ein- oder mehrfach durch C₁-C₄-Alkylgruppen substituiert ist und/oder einen kondensierten Phenylring aufweist.

11. Verbindung nach einem der Ansprüche 9 oder 10, worin
R⁴ für Wasserstoff steht und
R⁵ für gegebenenfalls substituiertes Phenyl steht.

12. Verbindung nach einem der Ansprüche 9 oder 10, worin R⁴ und R⁵ gemeinsam mit dem Kohlenstoffatom, an das sie gebunden sind, für einen 5- bis 10-gliedrigen gesättigten Carbocyclus stehen, der gegebenenfalls ein- oder mehrfach durch C₁-C₄-Alkylgruppen substituiert ist.

13. Verfahren zur Herstellung einer Pyrazolcarbonsäure der Formel la 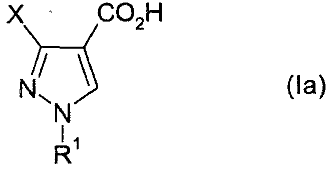 worin X und R¹ die in einem der Ansprüche 1 oder 5 angegebenen Bedeutungen aufweisen, umfassend die folgenden Schritte:
a) Bereitstellung einer Pyrazolverbindung der Formel I nach einem Verfahren gemäß einem der Ansprüche 1 bis 8,
b) Umwandlung der Verbindung I in eine 1,3-substituierte Pyrazolcarbonsäure der Formel la.

14. Verfahren zur Herstellung einer Verbindung der Formel VII 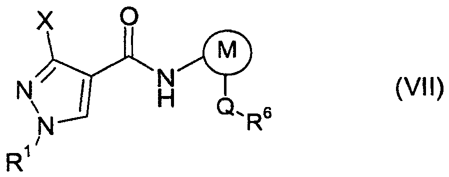 worin X und R¹ die in einem der Ansprüche 1 oder 5 angegebenen Bedeutungen aufweisen
M Thienyl oder Phenyl, das einen Halogensubstituenten tragen kann;
Q eine direkte Bindung, Cyclopropylen, ein annellierter Bicyclo[2.2.1]heptan- oder Bicyclo[2.2.1]hepten-Ring;
R⁶ Wasserstoff, Halogen, C₁-C₄-Alkyl, C₁-C₄-Halogenalkoxy, ein- bis dreifach substituiertes Phenyl, wobei die Substituenten unabhängig voneinander ausgewählt sind aus Halogen und Trifluormethylthio, oder Cyclopropyl;
umfassend die folgenden Schritte:
a) Bereitstellung einer Pyrazolverbindung der Formel I nach einem Verfahren gemäß einem der Ansprüche 1 bis 8,
b) Umwandlung der Verbindung I in eine 1,3-substituierte Pyrazolcarbonsäure der Formel Ia, 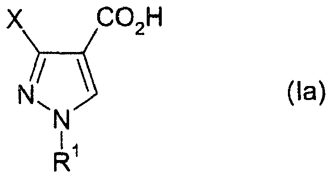 worin X und R¹ die zuvor angegebenen Bedeutungen aufweisen;
c) gegebenenfalls Überführung der Verbindung la in ihr Säurehalogenid, und
d) Umsetzung der Verbindung der Formel la oder ihres Säurehalogenids mit einer Aminverbindung der Formel VIII, 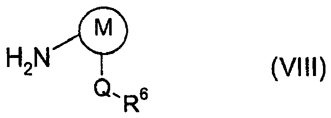 worin M, Q und R⁶ die für Formel VII angegebenen Bedeutungen aufweisen.

## Claims

1. A process for preparing 1,3,4-substituted pyrazole compounds of the formula I 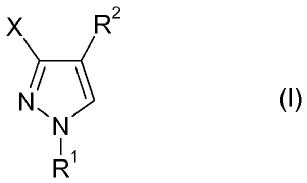 in which
X is a CX¹X²X³ group in which X¹, X² and X³ are each independently hydrogen, fluorine or chlorine, where X¹ may also be C₁-C₆-alkyl or C₁-C₄-haloalkyl and where at least one of the X¹, X² radicals is different from hydrogen,
R¹ is methyl, and
R² is a CO₂R^{2a} group in which R^{2a} is C₅-C₆-cycloalkyl, optionally substituted phenyl or C₁-C₆-alkyl which may optionally be substituted by C₁-C₄-alkoxy, phenyl or C₃-C₆-cycloalkyl,
comprising the following steps:
i) reacting a compound of the formula II with a hydrazone of the formula III 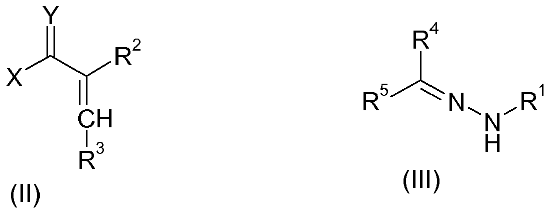 where the variables X and R² in formula II are each as defined for formula I,
Y is oxygen, an NR^{y1} group or an [NR^{y2}R^{y3}]⁺Z⁻ group, in which
R^{y1}, R^{y2} and R^{y3} are each independently C₁-C₆-alkyl, C₅-C₆-cycloalkyl, optionally substituted phenyl or optionally substituted phenyl-C₁-C₄-alkyl, or
R^{y2} and R^{y3} together with the nitrogen atom to which they are bonded are an N-bonded, 5- to 8-membered, saturated, optionally substituted heterocycle which, as well as the nitrogen atom, may also comprise 1 or 2 further heteroatoms selected from N, 0 and S as ring atoms, and Z⁻ is an anion;
R³ is OR^{3a} or an NR^{3b}R^{3c} group, in which
R^{3a}, R^{3b} and R^{3c} are each independently C₁-C₆-alkyl, C₅-C₆-cycloalkyl, optionally substituted phenyl or optionally substituted phenyl-C₁-C₄-alkyl, or
R^{3b} and R^{3c} together with the nitrogen atom to which they are bonded are an N-bonded 5-to 8-membered, saturated, optionally substituted heterocycle which, as well as the nitrogen atom, may also comprise 1 or 2 further heteroatoms selected from N, 0 and S as ring atoms,
and where the variable R¹ in formula III is as defined for formula I,
R⁴ and R⁵ are each independently hydrogen, C₁-C₆-alkyl which may optionally be substituted by C₁-C₄-alkoxy, phenyl or C₃-C₆-cycloalkyl, C₃-C₆-cycloalkyl or optionally substituted phenyl, where at least one of the R⁴ and R⁵ radicals is different from hydrogen, and where
R⁴ and R⁵ together with the carbon atom to which they are bonded may also be a 5- to 10-membered saturated carbocycle which is optionally mono- or polysubstituted by C₁-C₄-alkyl groups and/or optionally substituted phenyl, and/or comprises one or 2 fused phenyl rings;
ii) treating the reaction product obtained with an acid in the presence of water.

2. The process according to claim 1, additionally comprising the preparation of the compound III by reacting a carbonyl compound of the formula IV with a substituted hydrazine compound of the formula V 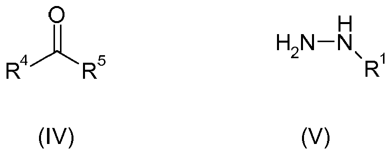 where R¹, R⁴ and R⁵ in the formulae IV and V are each as defined for formula III.

3. The process according to either of the preceding claims, in which R³ in formula II is O-R^{3a} in which R^{3a} is as defined above and is especially C₁-C₄-alkyl.

4. The process according to any one of the preceding claims, in which Y in formula II is oxygen.

5. The process according to any one of the preceding claims, where X in the formulae I and II is a CX¹X²X³ group in which X¹ and X² are each fluorine and X³ is hydrogen, fluorine or chlorine.

6. The process according to any one of the preceding claims, where R^{2a} in the COOR^{2a} group is C₁-C₆-alkyl or C₁-C₄-alkoxy-C₁-C₆-alkyl.

7. The process according to any one of the preceding claims, in which
R⁴ is hydrogen or C₁-C₆-alkyl and
R⁵ is C₁-C₆-alkyl, C₃-C₆-cycloalkyl or optionally substituted phenyl, or
R⁴ and R⁵ together with the carbon atom to which they are bonded may be a 5- to 10-membered saturated carbocycle which is optionally mono- or polysubstituted by C₁-C₄-alkyl groups and/or comprises a fused phenyl ring.

8. The process according to any of the preceding claims, in which
R⁴ is hydrogen and
R⁵ is optionally substituted phenyl.

9. A compound of the general formula VI 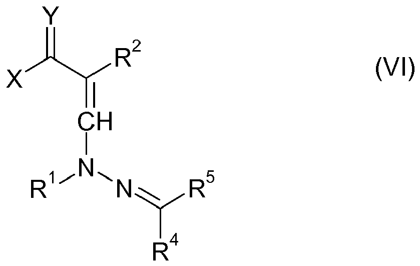 in which X, Y, R¹, R², R⁴ and R⁵ are each as defined in claim 1, excluding compounds of the formula VI in which R⁴ and R⁵ are each optionally substituted phenyl and Y is oxygen.

10. A compound according to claim 9, in which Y is oxygen;
X is a CX¹X²X³ group in which X¹ and X² are each fluorine and X³ is hydrogen, fluorine or chlorine;
R¹ is C₁-C₄-alkyl and especially methyl;
R² is a COOR^{2a} group in which R^{2a} is as defined in claim 1 and is especially C₁-C₆-alkyl or C₁-C₄-alkoxy-C₁-C₆-alkyl,
R⁴ is hydrogen or C₁-C₆-alkyl and
R⁵ is C₁-C₆-alkyl, C₃-C₆-cycloalkyl or optionally substituted phenyl, or
R⁴ and R⁵ together with the carbon atom to which they are bonded may be a 5- to 10-membered saturated carbocycle which is optionally mono- or polysubstituted by C₁-C₄-alkyl groups and/or comprises a fused phenyl ring.

11. A compound according to either of claims 9 and 10, in which
R⁴ is hydrogen and
R⁵ is optionally substituted phenyl.

12. A compound according to either of claims 9 and 10, in which
R⁴ and R⁵ together with the carbon atom to which they are bonded are a 5- to 10-membered saturated carbocycle which is optionally mono- or polysubstituted by C₁-C₄-alkyl groups.

13. A process for preparing a pyrazolecarboxylic acid of the formula Ia 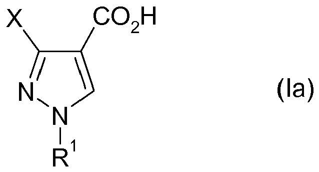 in which X and R¹ are each as defined in either of claims 1 and 5, comprising the following steps:
a) providing a pyrazole compound of the formula I by a process according to any one of claims 1 to 8,
b) converting the compound I to a 1,3-substituted pyrazolecarboxylic acid of the formula Ia.

14. A process for preparing a compound of the formula VII 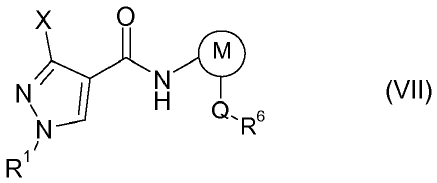 in which X and R¹ are each as defined in either of claims 1 and 5,
M is thienyl or phenyl which may bear a halogen substituent;
Q is a direct bond, cyclopropylene, a fused bicyclo[2.2.1]heptane or bicyclo[2.2.1]heptene ring;
R⁶ is hydrogen, halogen, C₁-C₄-alkyl, C₁-C₄-haloalkoxy, mono- to trisubstituted phenyl, where the substituents are each independently selected from halogen and trifluoromethylthio, or cyclopropyl;
comprising the following steps:
a) providing a pyrazole compound of the formula I by a process according to any one of claims 1 to 8,
b) converting the compound I to a 1,3-substituted pyrazolecarboxylic acid of the formula Ia, 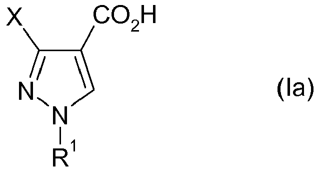 in which X and R¹ are each as defined above;
c) optionally converting the compound Ia to its acid halide, and
d) reacting the compound of the formula Ia or its acid halide with an amine compound of the formula VIII, 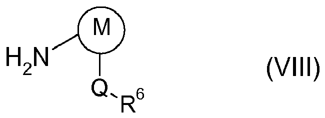 in which M, Q and R⁶ are each as defined for formula VII.

## Revendications

1. Procédé de fabrication de composés de pyrazole 1,3,4-substitués de formule I 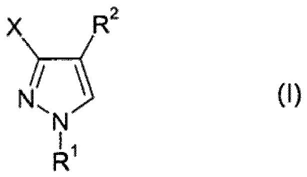 dans laquelle
X représente un groupe CX¹X²X³,
X¹, X² et X³ représentant indépendamment les uns des autres hydrogène, fluor ou chlore, X¹ pouvant également représenter alkyle en C₁-C₆ ou haloalkyle en C₁-C₄, et au moins un des radicaux X¹, X² étant différent de l'hydrogène,
R¹ représente méthyle, et
R² représente un groupe CO₂R^{2a},
R^{2a} représentant cycloalkyle en C₅-C₆, phényle éventuellement substitué ou alkyle en C₁-C₆, qui peut éventuellement être substitué par alcoxy en C₁-C₄, phényle ou cycloalkyle en C₃-C₆,
comprenant les étapes suivantes :
i) la mise en réaction d'un composé de formule II avec une hydrazone de formule III 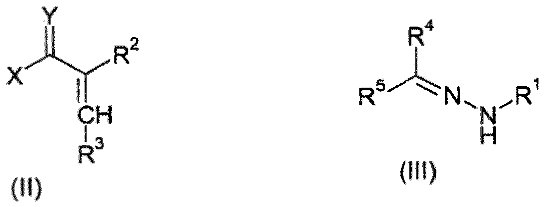 dans la formule II, les variables X et R² ayant les significations indiquées pour la formule I,
Y représentant l'oxygène, un groupe NR^{y1} ou un groupe [NR^{y2}R^{y3}] + Z⁻,
R^{y1}, R^{y2} et R^{y3} représentant indépendamment les uns des autres alkyle en C₁-C₆, cycloalkyle en C₅-C₆, phényle éventuellement substitué ou phényl-alkyle en C₁-C₄ éventuellement substitué, ou
R^{y2} et R^{y3} représentant ensemble avec l'atome d'azote auquel ceux-ci sont reliés un hétérocycle à liaison N, de 5 à 8 éléments, saturé, éventuellement substitué, qui, en plus de l'atome d'azote, peut également comprendre 1 ou 2 hétéroatomes supplémentaires choisis parmi N, 0 et S en tant qu'atomes de cycle, et
Z⁻ représentant un anion ;
R³ représentant OR^{3a} ou un groupe NR^{3b}R^{3c},
R^{3a}, R^{3b} et R^{3c} représentant indépendamment les uns des autres alkyle en C₁-C₆, cycloalkyle en C₅-C₆, phényle éventuellement substitué ou phényl-alkyle en C₁-C₄ éventuellement substitué, ou
R^{3b} et R^{3c} représentant ensemble avec l'atome d'azote auquel ceux-ci sont reliés un hétérocycle à liaison N, de 5 à 8 éléments, saturé, éventuellement substitué, qui, en plus de l'atome d'azote, peut également comprendre 1 ou 2 hétéroatomes supplémentaires choisis parmi N, 0 et S en tant qu'atomes de cycle,
et, dans la formule III, la variable R¹ ayant les significations indiquées pour la formule I,
R⁴ et R⁵ représentant indépendamment l'un de l'autre hydrogène, alkyle en C₁-C₆, qui peut éventuellement être substitué par alcoxy en C₁-C₄, phényle ou cycloalkyle en C₃-C₆ ; cycloalkyle en C₃-C₆ ou phényle éventuellement substitué, au moins un des radicaux R⁴ ou R⁵ étant différent de l'hydrogène, et
R⁴ et R⁵ pouvant également représenter ensemble avec l'atome de carbone auquel ils sont reliés un carbocycle saturé de 5 à 10 éléments, qui est éventuellement substitué une ou plusieurs fois par des groupes alkyle en C₁-C₄ et/ou phényle éventuellement substitué, et/ou qui comprend un ou 2 cycles phényle condensés ;
ii) le traitement du produit de réaction obtenu avec un acide en présence d'eau.

2. Procédé selon la revendication 1, comprenant en outre la fabrication du composé III par mise en réaction d'un composé de carbonyle de formule IV avec un composé d'hydrazine substitué de formule V 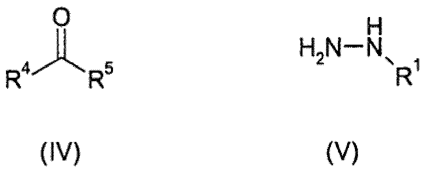 R¹, R⁴ et R⁵ dans les formules IV et V ayant les significations indiquées pour la formule III.

3. Procédé selon l'une quelconque des revendications précédentes, dans lequel R³ dans la formule II représente O-R^{3a}, R^{3a} ayant les significations indiquées précédemment et représentant notamment alkyle en C₁-C₄.

4. Procédé selon l'une quelconque des revendications précédentes, dans lequel Y dans la formule II représente l'oxygène.

5. Procédé selon l'une quelconque des revendications précédentes, dans lequel X dans les formules I et II représente un groupe CX¹X²X³, X¹ et X² représentant fluor et X³ représentant hydrogène, fluor ou chlore.

6. Procédé selon l'une quelconque des revendications précédentes, dans lequel R^{2a} dans le groupe COOR^{2a} représente alkyle en C₁-C₆ ou alcoxy en C₁-C₄-alkyle en C₁-C₆.

7. Procédé selon l'une quelconque des revendications précédentes, dans lequel
R⁴ représente hydrogène ou alkyle en C₁-C₆, et
R⁵ représente alkyle en C₁-C₆, cycloalkyle en C₃-C₆ ou phényle éventuellement substitué, ou
R⁴ et R⁵ représentent ensemble avec l'atome de carbone auquel ils sont reliés un carbocycle saturé de 5 à 10 éléments, qui est éventuellement substitué une ou plusieurs fois par des groupes alkyle en C₁-C₄ et/ou qui comprend un cycle phényle condensé.

8. Procédé selon l'une quelconque des revendications précédentes, dans lequel
R⁴ représente l'hydrogène et
R⁵ représente un phényle éventuellement substitué.

9. Composé de formule générale VI 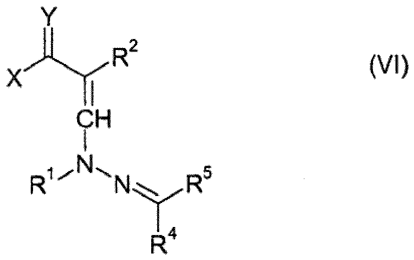 dans lequel X, Y, R¹, R², R⁴ et R⁵ ont les significations indiquées dans la revendication 1, à l'exception des composés de formule VI dans lesquels R⁴ et R⁵ représentent chacun phényle éventuellement substitué et Y représente oxygène.

10. Composé selon la revendication 9, dans lequel Y représente l'oxygène ;
X représente un groupe CX¹X²X³, X¹ et X² représentant fluor et X³ représentant hydrogène, fluor ou chlore ;
R¹ représente alkyle en C₁-C₄ et notamment méthyle ;
R² représente un groupe COOR^{2a}, R^{2a} ayant les significations indiquées dans la revendication 1 et représentant notamment alkyle en C₁-C₆ ou alcoxy en C₁-C₄-alkyle en C₁-C₆,
R⁴ représente hydrogène ou alkyle en C₁-C₆, et
R⁵ représente alkyle en C₁-C₆, cycloalkyle en C₃-C₆ ou phényle éventuellement substitué, ou
R⁴ et R⁵ représentent ensemble avec l'atome de carbone auquel ils sont reliés un carbocycle saturé de 5 à 10 éléments, qui est éventuellement substitué une ou plusieurs fois par des groupes alkyle en C₁-C₄, et/ou qui comprend un cycle phényle condensé.

11. Composé selon l'une quelconque des revendications 9 ou 10, dans lequel
R⁴ représente l'hydrogène et
R⁵ représente un phényle éventuellement substitué.

12. Composé selon l'une quelconque des revendications 9 ou 10, dans lequel
R⁴ et R⁵ représentent ensemble avec l'atome de carbone auquel ils sont reliés un carbocycle saturé de 5 à 10 éléments, qui est éventuellement substitué une ou plusieurs fois par des groupes alkyle en Cₗ-C₄.

13. Procédé de fabrication d'un acide pyrazole-carboxylique de formule la 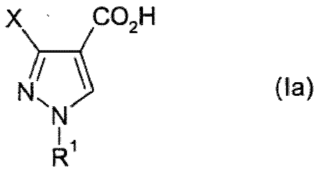 dans laquelle X et R¹ ont les significations indiquées dans l'une quelconque des revendications 1 ou 5, comprenant les étapes suivantes :
a) la préparation d'un composé de pyrazole de formule I par un procédé selon l'une quelconque des revendications 1 à 8,
b) la transformation du composé I en un acide pyrazole-carboxylique 1,3-substitué de formule Ia.

14. Procédé de fabrication d'un composé de formule VII 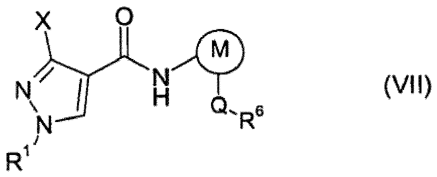 dans laquelle X et R¹ ont les significations indiquées dans l'une quelconque des revendications 1 ou 5,
M représente thiényle ou phényle, qui peut porter un substituant halogène ;
Q représente une liaison directe, cyclopropylène, un cycle bicyclo[2.2.1]heptane ou bicyclo[2.2.1]heptène annelé ;
R⁶ représente hydrogène, halogène, alkyle en C₁-C₄, halogénoalcoxy en C₁-C₄, phényle une à trois fois substitué, les substituants étant choisis indépendamment les uns des autres parmi halogène et trifluorométhylthio, ou cyclopropyle ;
comprenant les étapes suivantes :
a) la préparation d'un composé de pyrazole de formule I par un procédé selon l'une quelconque des revendications 1 à 8,
b) la transformation du composé I en un acide pyrazole-carboxylique 1,3-substitué de formule ia, 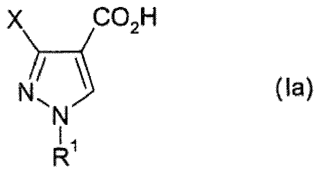 X et R¹ ayant les significations indiquées précédemment ;
c) éventuellement la transformation du composé Ia en son halogénure d'acide, et
d) la mise en réaction du composé de formule Ia ou de son halogénure d'acide avec un composé d'amine de formule VIII 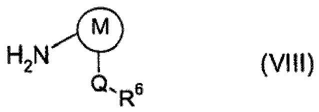 dans laquelle M, Q et R⁶ ont les significations indiquées pour la formule VII.
